# EUROPEAN PATENT APPLICATION

(11) **EP 4 736 874 A1**
(43) Date of publication of application: **06.05.2026**
(21) Application number: 24210633.4
(22) Date of filing: 04.11.2024
(51) Int. Cl.: A61K 40/11, A61K 40/42, C07K 14/475, C07K 14/555, A61K 40/31, C07K 16/28

(54) **METHODS FOR PREDICTING AND IMPROVING THE EFFICACY OF CAR T-CELL THERAPY**

(71) Applicant: Fondazione Matilde Tettamanti e Menotti de Marchi Onlus, 20900 Monza (MB) (IT); Universität Zürich, 8006 Zürich (CH)
(72) Inventor: MAGNANI, Chiara Francesca, CH 8006 Zürich (CH); PONZO, Marianna, CH-8006 Zürich (CH); GAIPA, Giuseppe, 20900 Monza (MB) (IT); BIONDI, Andrea, 20900 Monza (MB) (IT)
(74) Representative: Bianchetti & Minoja with Trevisan & Cuonzo IPS SRL

(57) **Abstract**

Disclosed is a treatment with a combination of inhibitors useful to improve the effectiveness of CAR T-cell therapies by inhibiting hypoxia, VEGF pathways and inflammation at the tumor microenvironment, thereby enhancing the response to CAR T-cell therapy in tumor patients. There is also disclosed a method for predicting the response to CAR T-cell therapy by determining T cell surface expression markers.

## Description

The present invention relates to a combination of biologically active agents useful to improve the effectiveness of CAR T-cell therapies. More precisely, the invention provides a combination of inhibitors able to counteract hypoxia, VEGF pathways and inflammation at the tumor microenvironment with CAR T-cell treatment thereby enhancing the response to CAR T-cell therapy in tumor patients. Another aspect of the invention relates to a method for predicting the response to CAR T-cell therapy by determining T cell surface expression markers.

### Background of the Invention

CAR T-cell therapy, targeting the CD19 antigen, has demonstrated promising efficacy in treating relapsed or refractory B-cell acute lymphoblastic leukemia (B-ALL). Nevertheless, only a fraction of B-ALL patients treated with CAR T cells maintains a durable remission long term. In a considerable portion of treated patients, relapse occurs in the presence of CD 19-expressing tumors, mainly because of CAR T-cell dysfunction and immune suppression within the tumor microenvironment (TME). Indeed, CAR T cells do not act on cancer cells in isolation, but within the TME, which is known to affect T-cell activity. Therefore, a major interest is to identify factors within the TME that influence the activity and potency of anti-CD19 CAR T cells with the final aim to develop more efficacious combinatorial therapies. We hypothesized that the immunological niche of the TME reacts to CAR T cell-mediated inflammation by modulating the immune response through the activation of inhibitory pathways and molecules, leading to insufficient CAR T-cell activity. Current CAR T-cell approaches are often insufficient in overcoming the TME barriers, as we demonstrated that the TME contains immunosuppressive factors, including myeloid-derived suppressor cells (MDSCs), hypoxia, and signaling molecules such as TGF-β, VEGF, type 1 and type 2 IFNs, and HIF1α. These factors promote T-cell dysfunction and interfere with CAR T-cell activity over time.

Prior attempts to improve CAR T-cell efficacy have not fully addressed the need to modulate these immunosuppressive factors within the TME. Therefore, a therapeutic solution is required to prevent or treat CAR T-cell dysfunction and T-cell exhaustion and to counteract the adverse effect of the TME. Moreover, there is a need for a biomarker that could predict poor response to CAR T-cell therapy and also stratify the patients potentially benefitting of combinatorial therapy.

### Description of the invention

In one aspect, the invention provides methods and compositions for enhancing CAR T cells-mediated cancer therapy by modulation of the hypoxic and/or chronically inflamed tumor microenvironment. In particular, the invention provides a therapeutic approach that combines targeting and inhibition of hypoxia-inducible and chronically inflamed factors, particularly HIF1α, VEGF and IFNs, to counteract TME-driven immunosuppression and improve the effectiveness of CAR T-cell therapy. By inhibiting the hypoxic and/or chronically inflamed tumor microenvironment, targeting HIF1α, VEGF, and IFN, the invention aims to reduce, in patients treated with CAR T-cell therapy, the immunosuppressive effect of the TME that contribute to T-cell exhaustion and CAR T-cell dysfunction, thereby maintaining a sustained anti-tumor response.

Accordingly, in a first embodiment the invention provides a HIF1α or VEGFR2 or IFN inhibitor, or a combination thereof, for use in the treatment of tumor by means of CAR T cells, wherein said inhibitors or combinations thereof enhance CAR T-cell activity by preventing T-cell exhaustion and by reducing the immunosuppressive effect of tumor microenvironment.

Tumors that can be treated according to the invention include stomach adenocarcinoma, colorectal carcinoma, hepatocellular carcinoma, gallbladder cancer, non-small cell bronchial carcinoma, non-small cell lung cancer, metastatic and malignant melanoma, breast cancer, pancreatic neuroendocrine tumor, soft tissue sarcoma, preferably hematological tumors and more preferably acute lymphoblastic leukemia (ALL).

The CAR T-cell therapy exploits cells that have been modified to express one or more engineered CAR molecules directed against a tumor antigen. Tumor antigens which can be targeted by CAR T-cells according to the invention include CD19, BAFFR, BCMA, BTLA, B7-H3, B7-H6, CAIX, CA9, CD20, CD22, CD72, CD30, CD33, CD38, CD44, CD44v6, CD70, CD123, CD33, CD117, ADGRE2, CLL-1, FLT3, SSTR2, CEA, CSPG4, EGFR, EGFR family including ErbB2 (HER2), EGFRvIII, EGP2, EGP40, ERBB3, ERBB4, ErbB3/4, EPCAM, EphA2, EpCAM, folate receptor-a, FAP, FBP, fetal AchR, GD2, CAIX, GD3, Glypican-2, Glypican-3 (GPC3), Her2, HLA-A1+MAGE1, HLA-A1+NY-ESO-1, IL-11Ralpha, IL-13Ralpha2, Lambda, Lewis-Y, Kappa, KDR, Melanoma-associated antigen (MAGE), MCSP, Mesothelin, Muc1, Muc16, NCAM, NKG2D Ligands, NY-ESO-1, Preferentially expressed antigen of melanoma (PRAME), PSC1, PSCA, PSMA, ROR1, SP17, Survivin, TAG72 TEMs carcinoembryonic antigen, HMW-MAA, AFP, CA-125, ETA, Tyrosinase, MAGE, laminin receptor, HPV E6, E7, BING-4, Calcium-activated chloride channel 2, Cyclin-B 1, 9D7, EphA3, Telomerase, SAP-1, BAGE family, CAGE family, GAGE family, MAGE family, SAGE family, XAGE family, NY-ESO-1/LAGE-1, PAME, SSX-2, Melan-A/MART-1, GP100/pmel17, TRP- 1/-2, P. polypeptide, MC1R, Prostate-specific antigen, b-catenin, BRCA1/2, CML66, Fibronectin, MART-2, TGF-bRII, or VEGF receptors (e.g., VEGFR2).

In a preferred embodiment, anti-CD19 CAR T cells and CARCIK-CD19 cells are used. The CD19 CARs may include one or more costimulatory domains, such as CD28, 4-1BB, OX40, CD27, ICOS, or a combination thereof. The CAR molecule may include one or more transmembrane domains, such as CD28, CD8alpha, CD3-zeta, CD4, and a combination thereof. In specific embodiments, the T cells may be CD4+ T cells, CD8+ T cells, CARCIK cells, Treg cells, Th1 T cells, Th2 T cells, Th17 T cells, gamma delta T cells, unspecific T cells, or a population of T cells that comprises a combination of any of those mentioned.

The HIF1α, VEGFR2 and/or IFN inhibitors can be used singularly, in different binary combinations or as a ternary combination. In one embodiment, the binary combination includes HIF1α with VEGFR2 inhibitors.

The efficacy of the cell therapy is enhanced by the use of one or more inhibitors and the combinatorial approach provides greater anti-tumor activity compared to using the components of the combination separately.

Inhibitors of HIF1A include PX-478, EZN-2968, Topotecan, Digoxin, Acriflavine, inhibitors of HIF-PHD, such as roxadustat, vadadustat, daprodustat, molidustat, desidustat; inhibitors of HIF2A, such as PT2385 / PT2977 (Belzutifan); inhibitors of VEGFR2/VEGFs.

Inhibitors of VEGFR2 include anti-VEGFR2 antibodies, such as Ramucirumab; Tyrosin kinase inhibitor targeting VEGFR2, such as Sunitinib, Axitinib, Cabozantinib, Sorafenib, Dasatinib, Nilotinib, Pazopanib, Regorafenib; anti-VEGF antibodies or decoy receptors, such as Bevacizumab (Avastin), Aflibercept (Zaltrap), Ranibizumab.

IFN inhibitors include interferon gamma (IFNγ)-blocking antibodies, such as Emapalumab (Gamifant); type I interferon (IFN) receptor antagonist, such as Anifrolumab; IFN-alpha inhibitors, such as chloroquine, bafilomycin, chlorpromazine, inhibitors of VEGFR2/VEGFs

Preferably the inhibitors are selected from the group consisting of PX-478 (1) Ramucirumab (2), Anifrolumab (3) and Emapalumab (4).

As part of the therapy, the T cells are used in conjunction with one or more particular inhibitors or targeting antibodies. In such cases, they may be administered or otherwise provided simultaneously, e.g. in the same medicament, separately - i.e. in separated medicaments administered one after the other - or sequentially in any order. Sequential administration is particularly useful when the therapeutic agents are in different dosages forms (e.g. one agent is a tablet or capsule and another agent is a sterile liquid) and/or are administered with different dosing schedules, e.g., one agent that is administered at least daily, once weekly, once every two weeks, or once every three weeks and another agent is administered less frequently, such as once, twice or with multiple infusions, daily, once weekly, once every two weeks, or once every three weeks.

The VEGFR, HIF1α and IFN inhibitors or targeting antibodies in a combination therapy with CAR-T cells can be administered immediately before, during or immediately after administration of the CAR T cell therapy, where by "immediately before or after" it is intended a time period from the enrollment to 12 months post CAR T-cell infusion, preferably from 1 month before the infusion to 6 months after the infusion.

The therapeutic agents in the combination therapy can be delivered using the same dosing regimen (dose, frequency, and duration of treatment) as would normally be used when the active ingredient is used as monotherapy for the treatment of the same cancer.

Alternatively, the patient receives a lower total amount of at least one of the therapeutic agents in the combination therapy than in the case of monotherapy, for example lower dosages, less frequent dosing and/or a shorter treatment period.

Any therapeutic agent in a combination therapy of the invention may be administered orally or parenterally, including intravenously, intramuscularly, intraperitoneally, subcutaneously, rectally, topically and transdermally. Preferably, the inhibitor is administered at a dosage that effectively reduces the hypoxic stress and VEGF signaling without causing excessive toxicity to the CAR T cells.

In a preferred embodiment, the biotherapeutic agents in a combination therapy of the invention are administered by continuous infusion, or by doses at intervals of, e.g., daily, every other day, three times per week, or one time each week, two weeks, three weeks, monthly or bimonthly. A total weekly dose is generally at least 0.05 µg/kg, 0.2 µg/kg, 0.5 µg/kg, 1 µg/kg, 10 µg/kg, 100 µg/kg, 0.2 mg/kg, 1.0 mg/kg, 2.0 mg/kg, 10 mg/kg, 25 mg/kg, 50 mg/kg per body weight or more.

In one embodiment, CAR T-cell therapy in a combination therapy of the invention may be generated in vivo by administration of a vector intravenously, intramuscularly, intraperitoneally, subcutaneously, rectally, topically and transdermally. The combination therapy of the invention may be used prior to or following lymphodepletion, prior to or following surgery to remove a tumor and prior to, during or after radiation therapy.

In the combination therapy according to the invention, the dosing regimen will comprise administering CAR T cells at a dose of 0, 1, 2, 3, 5, 10 or 15 millions cells/kg. In certain embodiments, a subject will be administered an intravenous (IV) infusion of a medicament comprising any of the CAR T cells described herein. In one preferred embodiment of the invention, the CAR T cell therapy in the combination therapy is CARCIK-CD19, which is administered intravenously at a dose consisting of 7.5 million cells/Kg. In another preferred embodiment, the CAR T cell therapy in the combination therapy is a commercial and investigational CAR T cell therapy, which is administered intravenously at a dose consisting of 2-5 million cells/Kg.

In some embodiments, a combination therapy of the invention is administered to a patient who is treatment-naive, not having received prior treatment. In other embodiments, the combination therapy is administered to a patient who is treatment-experienced and has not achieved a sustained response after therapy with a biotherapeutic or chemotherapeutic agent, including one of the agents of the combination therapy. In some embodiment, the clinical achievement by the combination therapy of the invention is any increase in complete remission (CR), partial remission (PR), DOR, PFS, EFS, OS.

The combination therapy of the invention is typically used for the treatment of tumors that are relapsed or refractory to previous treatments. In preferred embodiments, a combination therapy of the invention is used to treat an advanced stage tumor, as primary refractory diseases, or first relapse within 12 months of 1^{st} line therapy, or relapse / refractory following at least 2 lines of prior therapy, or relapse / refractory following at least 100 days after allogeneic Stem cell transplantation (allo-SCT), or dimensions of at least about 200 mm³, 300 mm³, 400 mm³, 500 mm³, 750 mm3, or up to 1000 mm³.

In another aspect, the invention provides a method that allows the identification of patients that are poorly responsive to CAR T-cell therapy and that can benefit from CAR T-cell therapy in combination with targeting and inhibition of the hypoxia-inducible and chronically inflamed factors HIF1α, VEGF and IFN.

This aspect of the invention is based upon the finding of endogenous T cells not expressing the CAR and expressing particular exhaustion cell surface biomarkers that allow to predict responsiveness to CAR T-cell therapy and to stratify patients associated with poor response after CAR T-cell therapy. While some predictors of poor duration of response or limited survival are known in the art, there is, until now, no biomarker able to stratify patients affected by the immunosuppressive TME post CAR-T cell treatment. The ability to use a biomarker to predict poor duration of response (DOR), limited progression-free survival (PFS), event-free survival (EFS), or overall survival (OS) of CAR T-cell therapy response allows for identifying and stratifying patients that could benefit from improved combinatorial methods with the aim of increasing the activity of CAR T-cell immunotherapy.

Preferably, a T-cell surface marker enabling the identification of poor responsive cancers post CAR T-cell therapy is selected from the group consisting of PD1, TIGIT, TIM3 and LAG3.

Accordingly, the invention provides a method of predicting the response to CAR T-cell therapy, which comprises determining, in a sample of T cells isolated from a subject undergoing CAR T-cell therapy, the expression of a cell surface marker selected from PD1, TIGIT, TIM3 and LAG3, or a combination thereof, wherein detection of said surface marker expression in the T cell sample is indicative of a poor response to the CAR T-cell therapy. Detection of the biomarker PD-1 is preferred.

The identification of patients poorly responsive to CAR T-cell therapy allows to drive the CAR T cell-based combinatorial therapy according to the invention. In particular, a combination therapy of the invention is preferably administered to a human patient who has T cells that tests positive for one or more of TIGIT, TIM3, LAG3, preferably PD1 expression post CAR T-cell treatment. In a preferred embodiment, PD1 expression is detected using diagnostic anti-human PD1 antibody or antigen-binding fragment thereof in FACS analysis or IHC analysis of FFPE or frozen tissue section of the tumorigenic sample. Typically, the patient's physician orders a diagnostic test to determine PD1 expression in a tumor tissue sample removed from the patient prior to initiating treatment with the HIF1α, VEGF, and IFN inhibitors, but it is envisioned that the physician could order the first or subsequent diagnostic tests any time after initiating the treatment, such as after completion of a treatment cycle.

### Description of the Figures

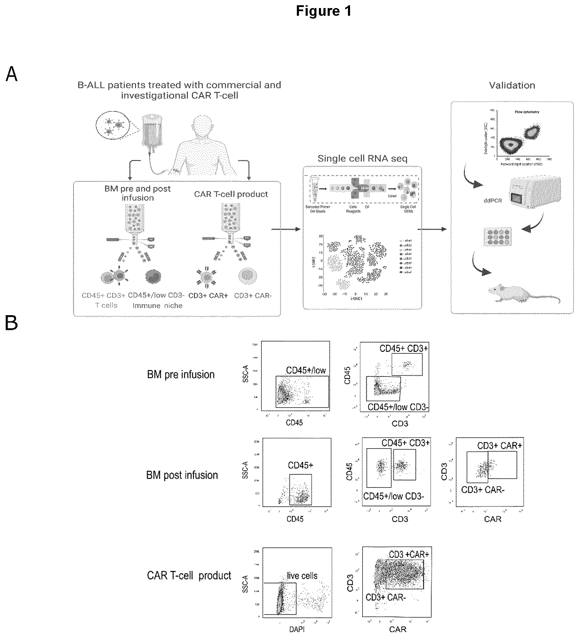

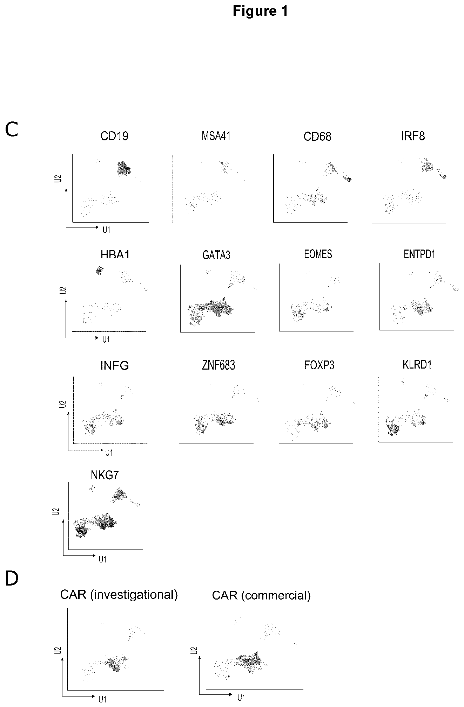

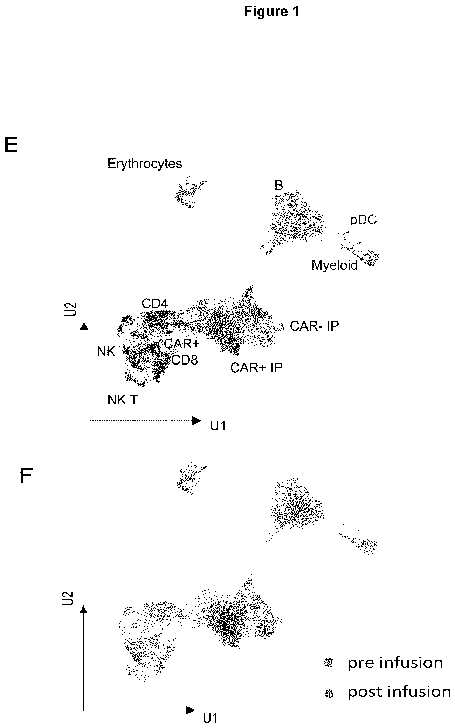

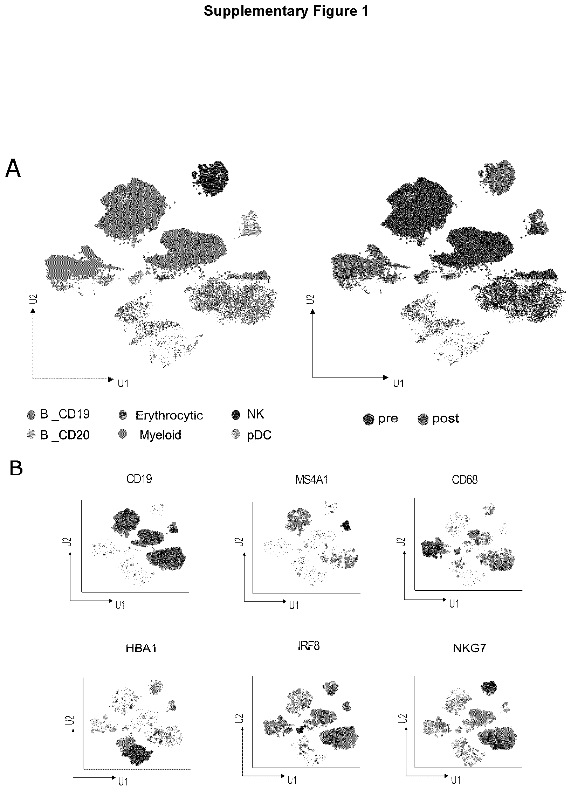

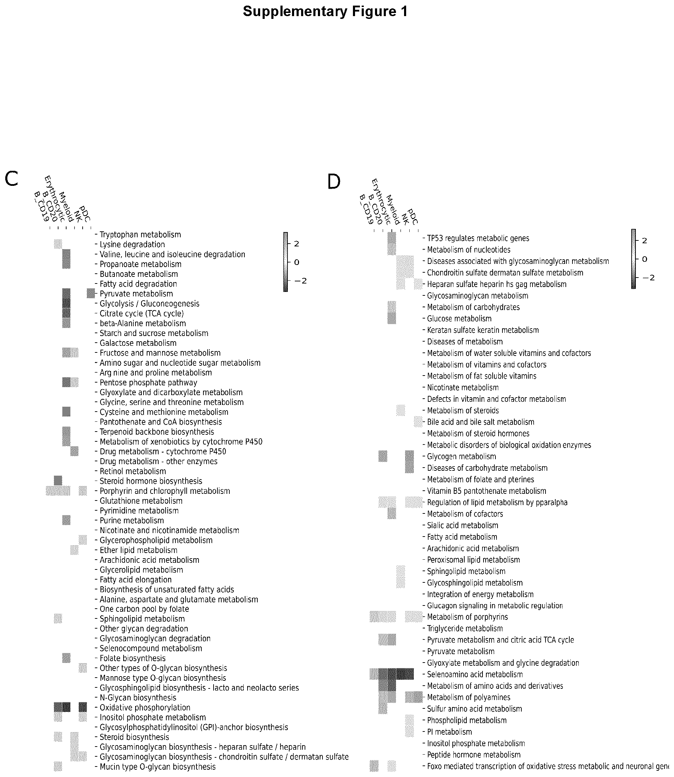

**Figure** 1. Single-cell RNA sequencing unveils profound restyling in the TME post CAR T-cell treatment. (A) Experimental design and sorting strategy. Sequencing libraries have been generated from BM samples of pediatric B-ALL patients before and after anti-CD19 CAR T cell infusion, and the infusion product (IP). Cells were sorted and subjected to scRNA-seq. Data were subsequently validated using spectral flow cytometry, ddPCR, in vitro co-cuture and in vivo humanized models. (B) Representative flow cytometry gating applied for sorting B-ALL BM samples and the IP. (C) Expression levels of lineage-specific genes overlaid on the Uniform Manifold Approximation and Projection (UMAP), (D) Transcript-based CAR T-cell cluster identification analysis. (E-F) UMAP embedding of multidimensional scRNA-seq data by cell clusters and time points.

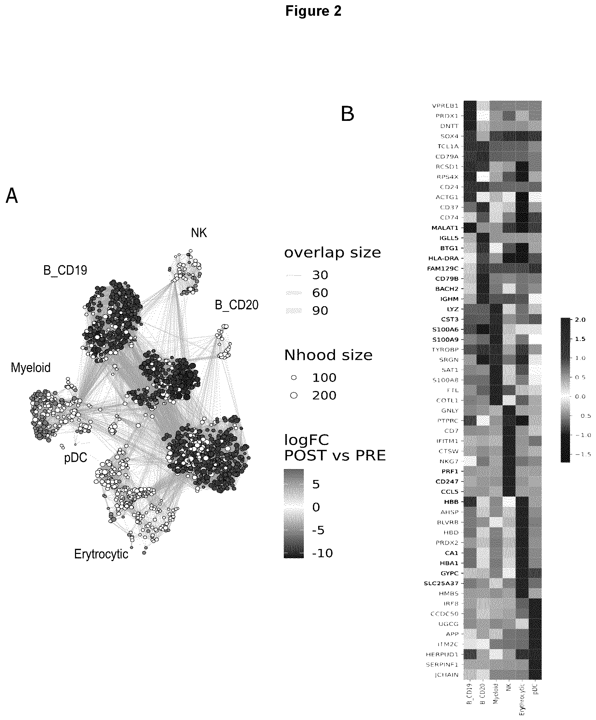

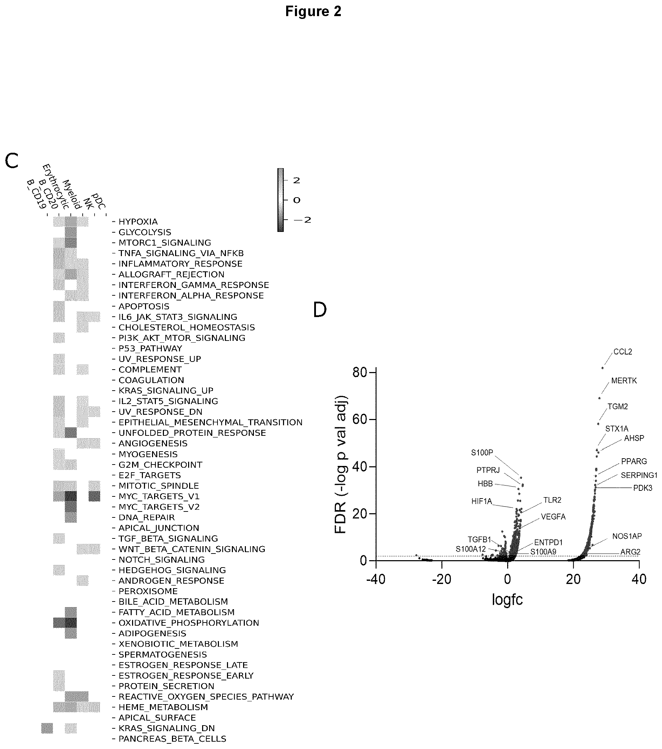

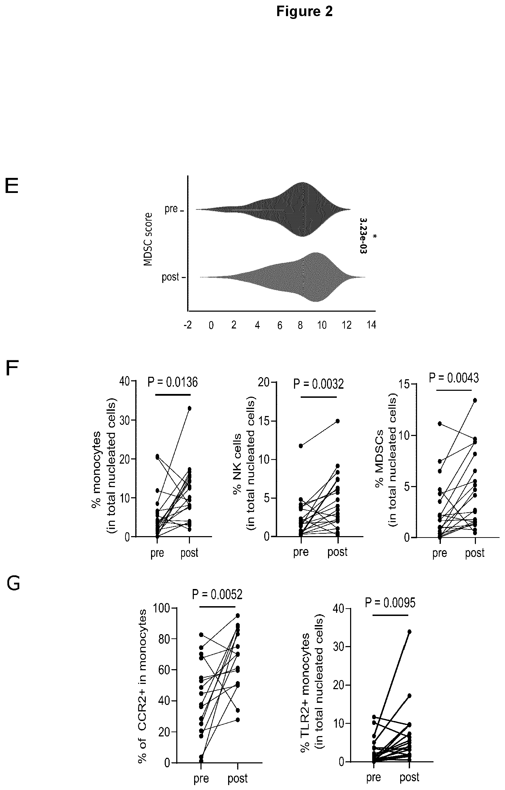

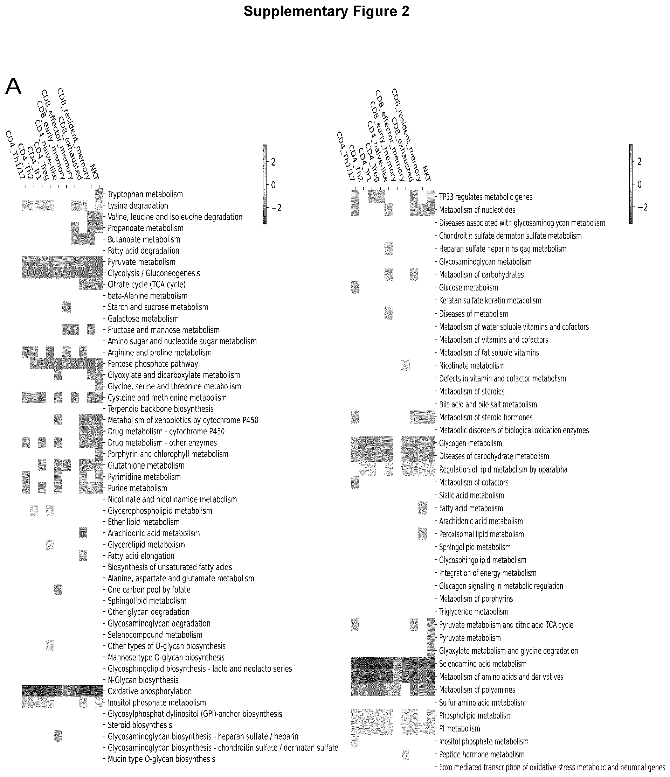

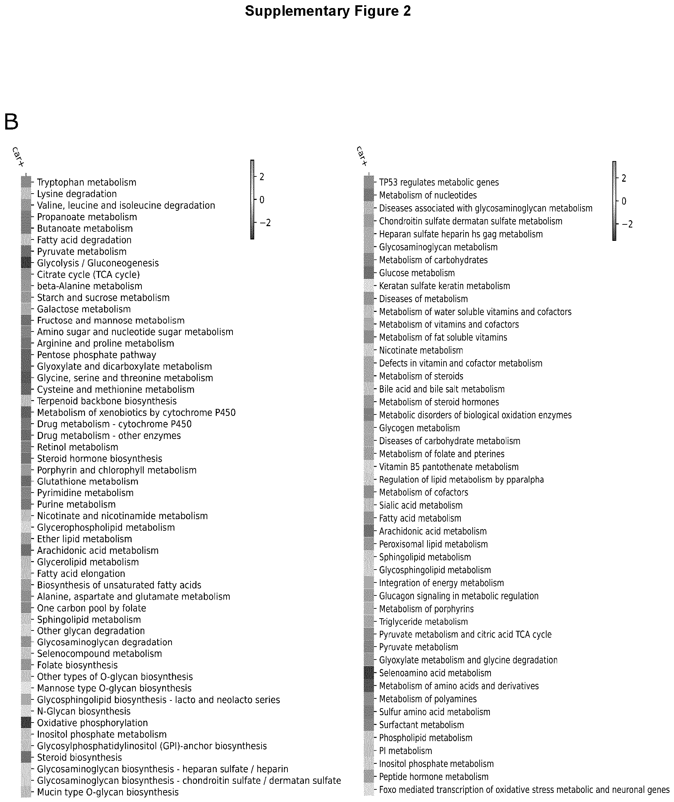

**Figure 2****.** Bone Marrow Myeloid cells are enriched in immunosuppressive Myeloid-Derived Suppressor Cells after CAR T-cell injection. (A) A neighborhood graph of the differential abundance test comparing samples post-infusion to pre-infusion with MILO and neighborhoods displaying significant abundance are expressed as log fold change (logFC). Size corresponds to the number of cells in each neighborhood (Nhood) in the UMAP space. Edge width (overlap size) is proportional to the number of cells shared between neighborhoods. As expected, limited overlap is observed between different cell types. (B) Gene expression Heatmap of the top ten cell-type-specific marker genes. (C) Significantly-enriched Molecular Signatures Database Hallmark gene sets in post-treatment versus pre-treatment within each immune cell types of the CD45+CD3- libraries (adjusted P val< 0.05). (D) Volcano Plots of DEG in myeloid cell cluster indicating genes up-regulated (rightward direction) and genes down-regulated (leftward direction) in post-CAR T-cell infusion compared to pre infusion. (E) MDSC signature scoring at different time points (arbitrary unit). (F) Flow cytometry analysis (n=20 paired pre- and post- treatment samples) with Infinicyt depicting frequencies of monocytes (CD33+CD14+ or CD16+), NK (CD3-CD56+) and MDSC (CD11b+CD33+HLA-DRlowCD14-CD15+ or CD14+CD15-) in total nucleated cells. Statistical analysis was performed using Wilcoxon-matched paired t- test. (G) Flow cytometry analysis depicting the frequencies of CCR2+ cells gated in monocytes and the frequencies of TLR2+ monocytes in total nucleated cells.
**Supplementary** **Figure 1****.** CD45+CD3- compartment analysis in CAR T-cell treated samples versus untreated. (A) UMAP visualization of the CD45+CD3- libraries as cell clusters (left) and time points (right). (B) Marker-based cell type identification analysis allowed prediction of six broad immune cell types across all profiled single cells in CD45+CD3-fraction. (C-D) Significantly enriched KEGG (left) and Reactome (right) gene sets in post-treatment versus pre-treatment samples within each of the immune cell types.

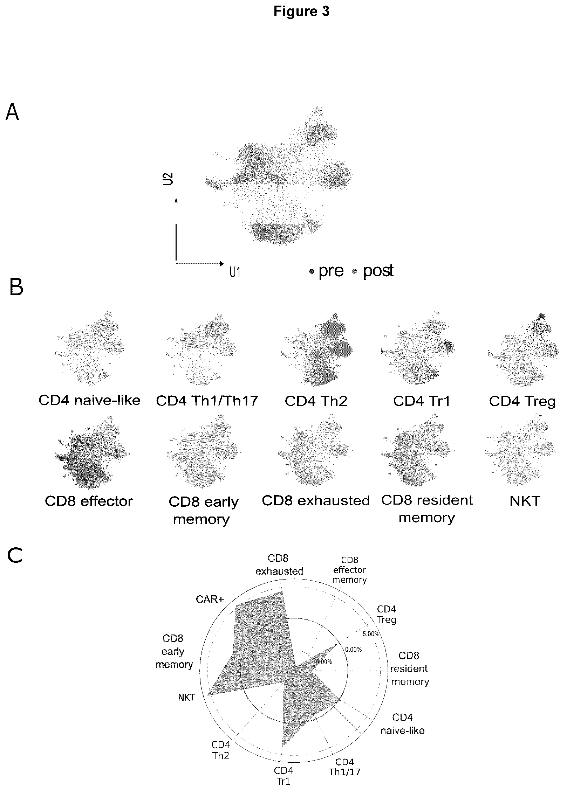

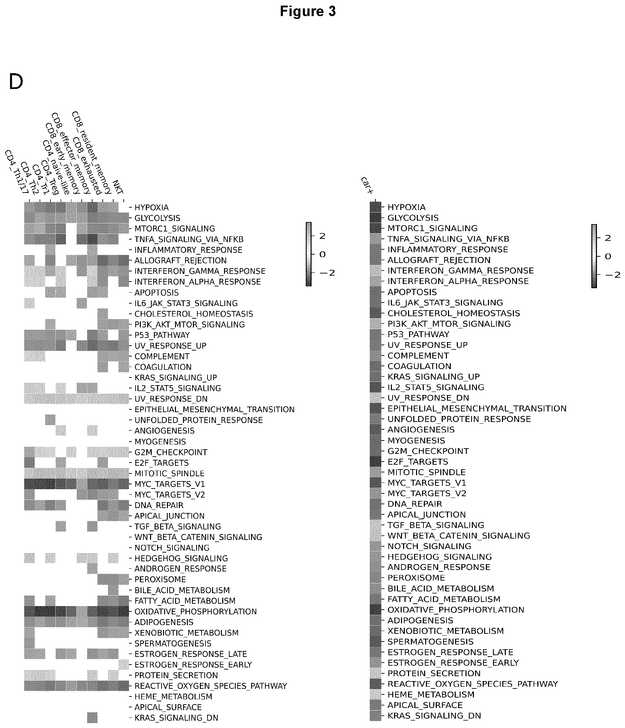

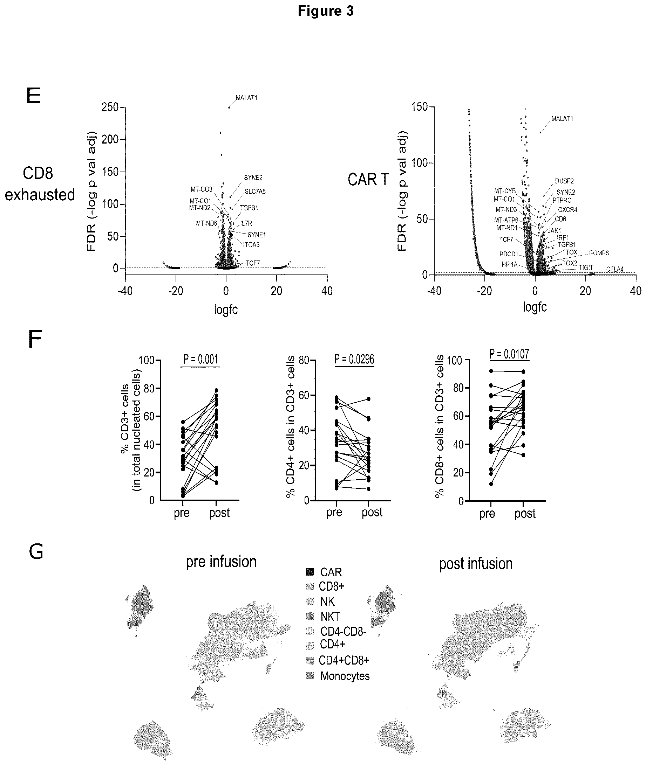

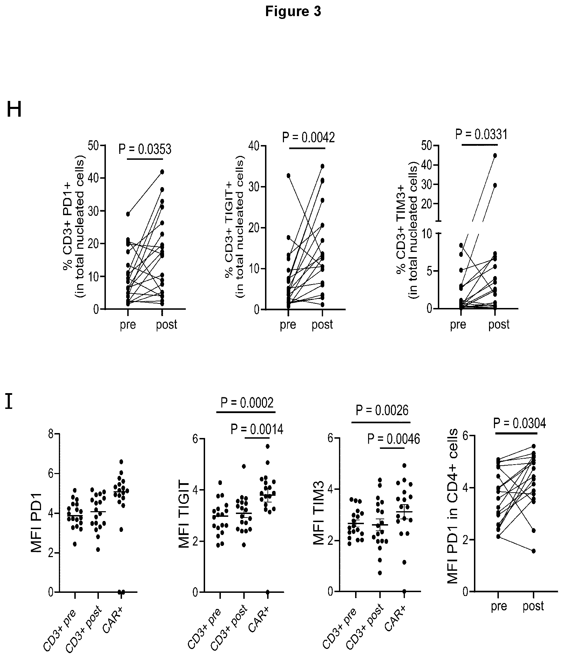

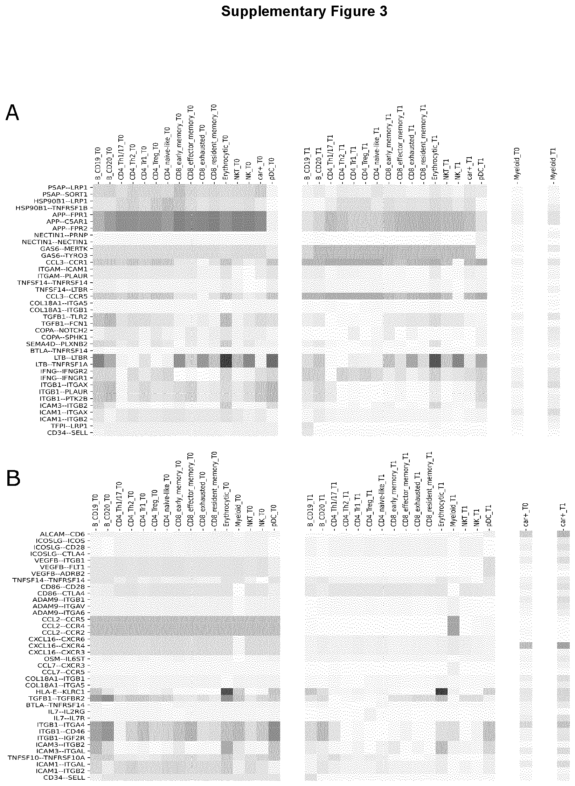

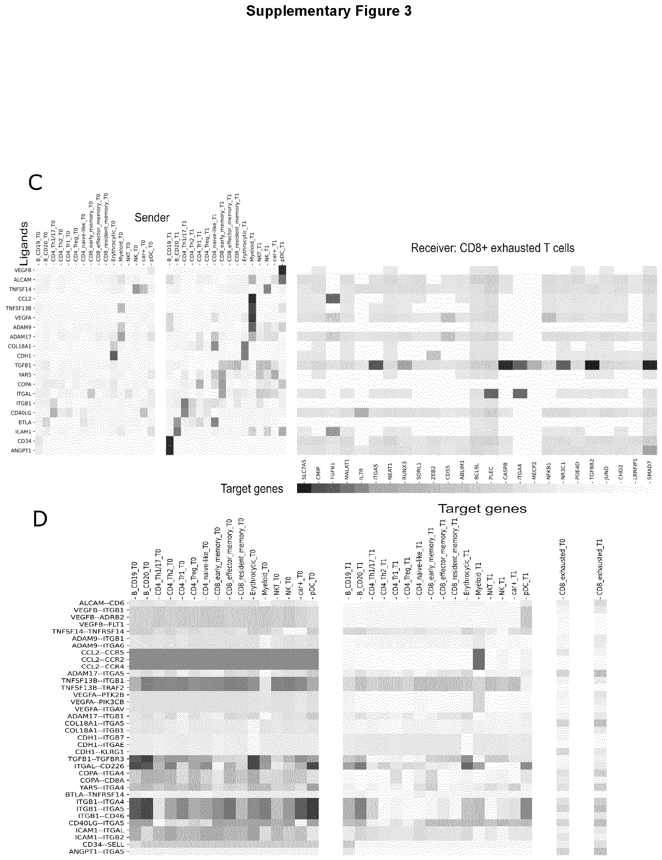

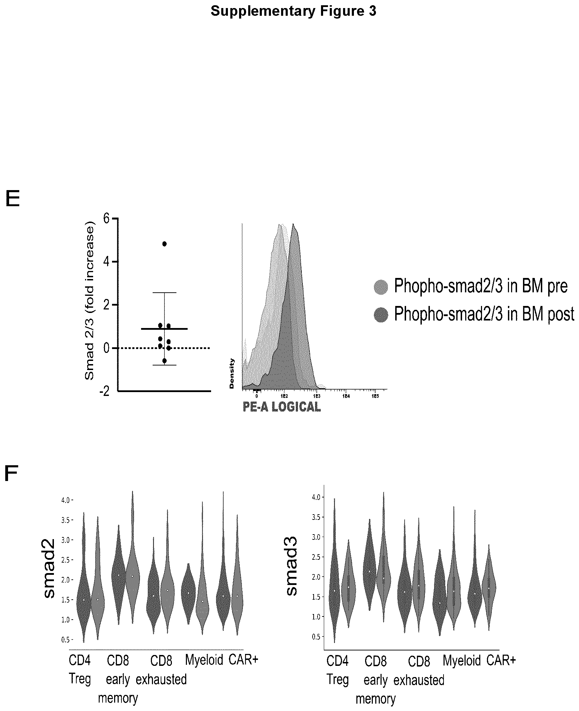

**Figure 3****.** The TME post CAR T-cell treatment is characterized by Increased T-cell Exhaustion. (A) UMAP visualization of individual cells in the CD45+CD3+ libraries before and after anti-CD19 CAR T-cell treatment. (B) Garnett cluster-extended type classification of the CD3+ subpopulations. (C) Relative frequency of CD45+CD3+ cell subtypes. Delta proportion to estimate abundance reveals an increase in exhausted CD8+ T cells, CD4+ Type 1 regulatory T cells, and NKT after CAR T-cell treatment. (D) Significantly-enriched Molecular Signatures Database Hallmark gene sets in post-treatment versus pre-treatment CD45+CD3+ libraries within each immune cell types (left) and in CAR T cells (right). (E) Volcano Plots of differentially expressed genes (DEGs) in CD8 exhausted cell cluster (up) and CAR T cells (below) indicating genes up-regulated (rightward direction) and genes down-regulated (leftward direction) post CAR T-cell infusion compared to pre infusion. (F) Flow cytometry analysis (n=20 pre- and n=20 post- treatment paired BM samples) with Infinicyt depicting the frequencies of CD3+ cells in total nucleated cells and of CD4+ and CD8+ in CD3+ cells. Wilcoxon matched-pair t test. (G) UMAP representation with FlowSOM overlay of randomly selected cells (55000 per file) from 36 paired samples comparing before and after treatment. (H) Flow cytometry analysis with Infinicyt depicting frequencies of CD3+PD1+, CD3+TIGIT+, CD3+TIM3+ cells in total nucleated cells. Wilcoxon matched-pairs signed-rank test. (I) FlowSOM-generated MFI analysis of PD1, TIGIT and TIM3 in CD3+ cells before and after treatment and in CAR T cells. Statistical analysis was performed using one-way ANOVA with Friedman multiple comparison. MFI of PD1 in CD4+ cells before and after treatment. Statistical analysis performed using Wilcoxon matched-pairs signed-rank t test.
**Supplementary** **Figure 2****.** Endogenous T cells and infused CAR T cells showed increased lipid metabolism post treatment. (A-B) Significantly enriched KEGG (left) and Reactome (righ) gene sets in post-treatment versus pre-treatment samples within each of the A) endogenous CD3+ immune cell types (top) and B) within CAR T cells (below).

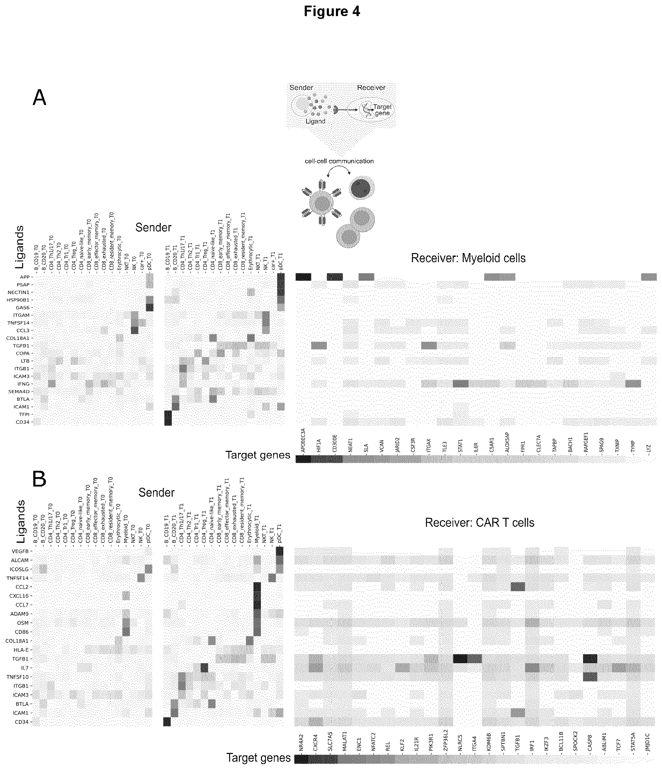

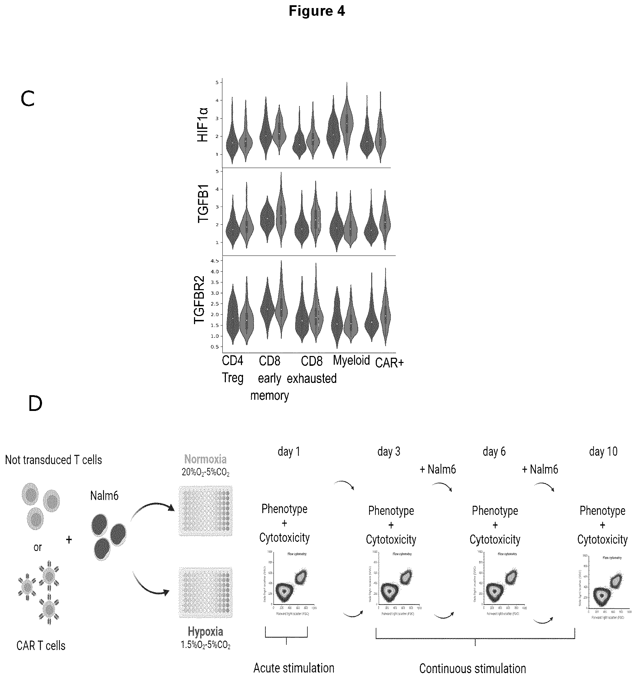

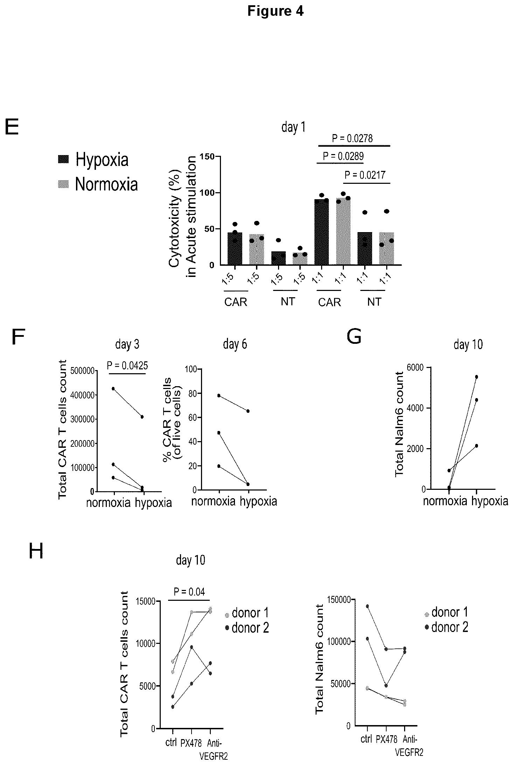

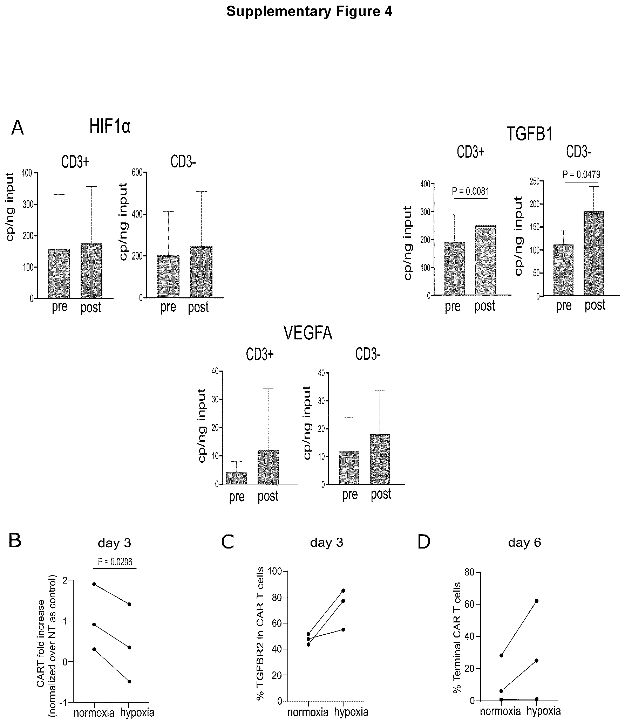

**Figure 4****.** NicheNet-inferred cell-to-cell interactions revealed a role of chronic inflammation and hypoxia in driving T cell dysfunction, which can be reversed by pharmacologic treatment with HIF1α inhibitor (PX478) and anti-VEGFR2 antibodies. (A) NicheNet's ligand activity prediction (left) and ligand-target matrix (right) assessed using all niche as sender and myeloid compartment as receiver and (B) using all niche as sender and CAR T cells as receivers. Heatmap visualization of prioritized ligands' gene expression levels (Z-score) in specific cell compartments of all niche (left) and of predicted ligand activity in the receiver population (right). (C) Violin plots of scRNA-seq data depicting HIF1α, TGFB1 and TGFBR2 expression distribution in different immune cell clusters before and after treatment. (D) Schematic outline of the co-culture experiment simulating acute and chronic stimulation under normoxic and hypoxic conditions, in which CAR T cells are stimulated with Nalm6 cells for 24 hours (acute) or repeatedly every 3 days (chronic) at an effector:target (E:T) ratio of 1:1. (E) Cytotoxicity of CAR T cells after 24 hours of co-culture with Nalm6 cells at different effector to target (E:T) ratios compared with not-transduced (NT) cells (left). One-way ANOVA with Tukey multiple comparison. Data in E illustrate the mean ± SD of triplicates from three different donors. Paired t test. (F) Flow cytometry analysis depicting the CAR T-cell count and frequency after 3 and 6 days of co-culture, respectively. (G) Flow cytometry analysis depicting the outgrowing Nalm6 cell count after 10 days of co-culture. Data in F-G illustrate the mean of triplicates from three different donors. Paired t test. (H) Flow cytometry analysis depicting the CAR T-cell count and outgrowing Nalm6 cell count after 10 days of co-culture in the absence or presence of HIF1α inhibitor (PX478) or anti-VEGFR2 antibodies at an E:T ratio of 1:3. Data are presented as duplicates from two different donors. P value is determined by paired non parametric test (Friedman test with multiple comparison).
**Supplementary** **Figure 3****.** NicheNet-inferred ligand-receptor interactions revealed an autocrine TGF-β signaling loop in CAR T cells and endogenous T cells. (A-B) NicheNet Heatmap depicting potential ligand-receptor link activities assessed using A) all niche as sender and myeloid compartment as receiver and B) using all niche as sender and CAR T cells as receivers. (C) NicheNet's ligand activity prediction (left) and ligand-target matrix (right) assessed using all niche as sender and CD8+ exhausted T cells as receiver. (D) NicheNet Heatmap depicting potential ligand-receptor link activities assessed using all niche as sender and CD8+ exhausted T cells as receiver. (E) Phospho-flow analysis of smad2/3 phosphorylation in CD3+ cells assessed by intracellular staining. Data illustrate the mean ± SD of the fold increase expression in BM pre-treatment compared to post-treatment. One representative patient of results in 8 paired samples is shown. (F) Violin plots of scRNA-seq data depicting smad2 and smad3 expression distribution in different immune cell clusters before and after treatment
**Supplementary** **Figure 4****.** Chronic antigen stimulation in hypoxic conditions promotes T-cell dysfunction. (A) Flow cytometry analysis depicting CAR T-cell fold increase in hypoxic compared to normoxic condition after 3 days of co-culture. (B) Flow cytometry analysis depicting the frequencies of TGFBR2+ CD3+ cells after 3 days of co-culture. (C) Flow cytometry analysis depicting the frequencies of terminal CAR+ cells after 6 days of co-culture. Data illustrate the mean ± SD of triplicates from three different donors. Wilcoxon matched-pair t test. *, P < 0.05. (D) ddPCR validation of HIF1α, TGFB1 and VEGFA genes in purified CD3+ and CD3- cell subsets (n=13 paired pre- and post- BM treatment samples). Data illustrate the mean ± SD. Wilcoxon matched-pair t test.

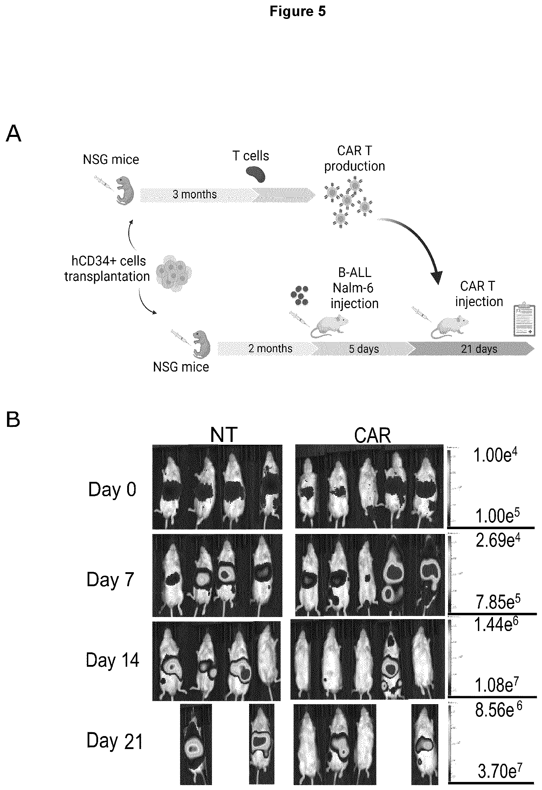

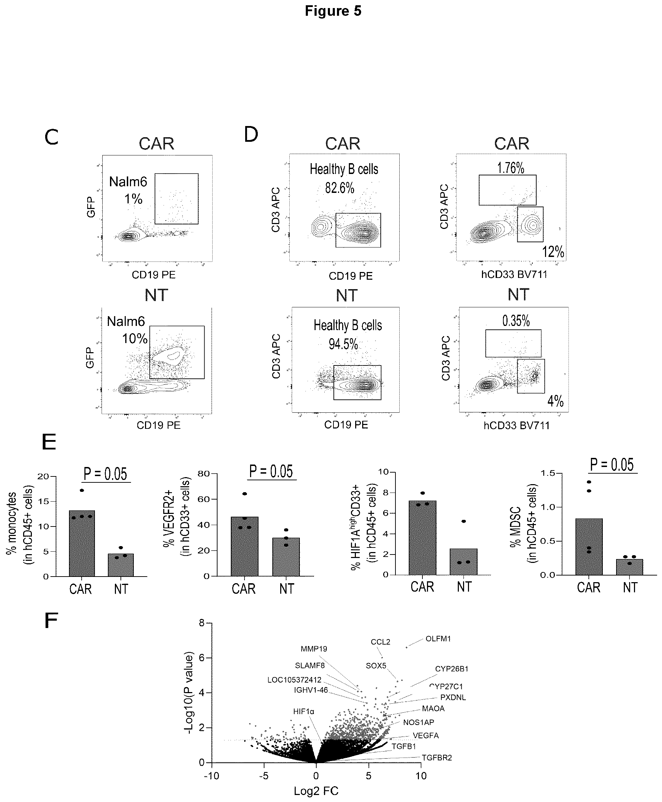

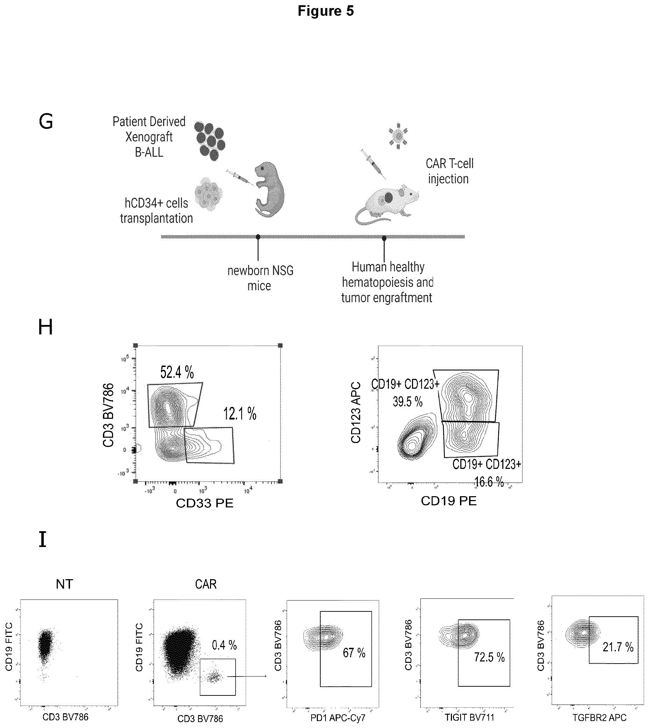

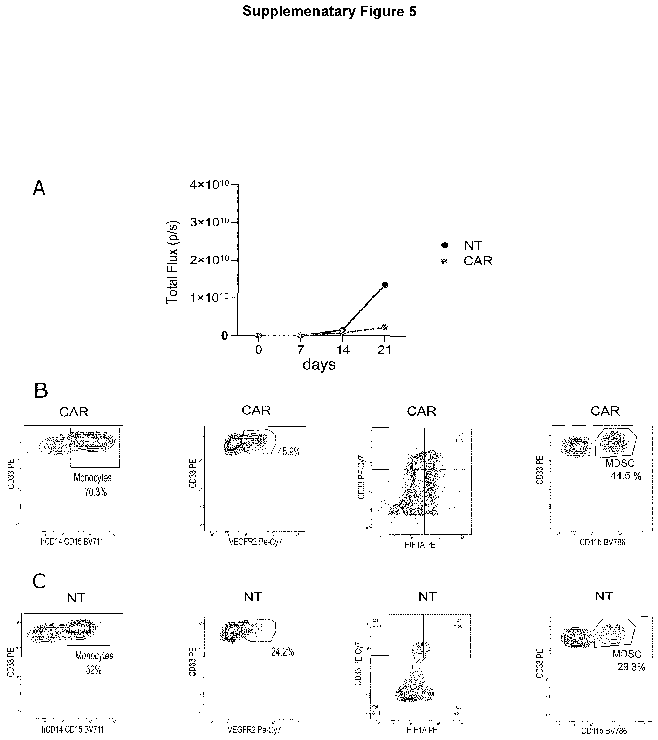

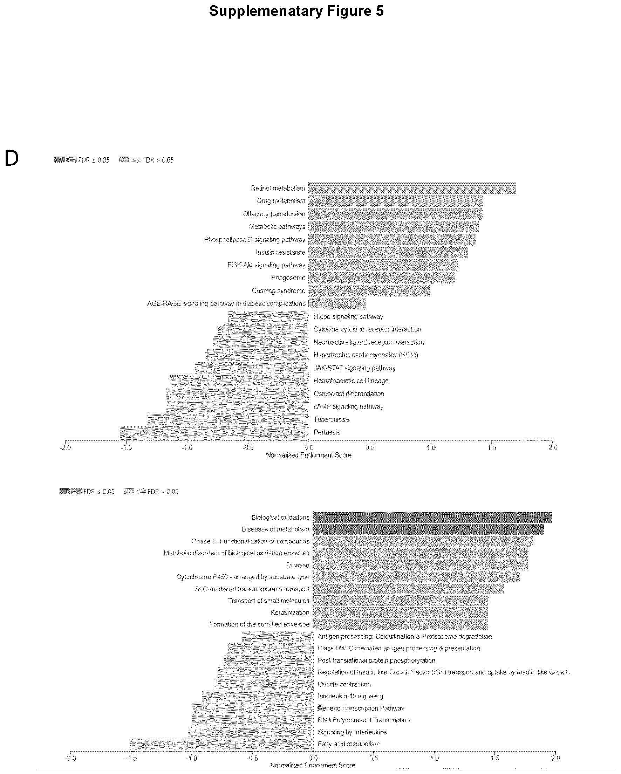

**Figure 5****.** CAR T cells treatment remodeled the TME in tumor-bearing HSPC-humanized mice. (A) Newborn NSG mice were sub-lethally irradiated, injected intrahepatically with human PB-mobilized CD34+ cells and, after establishment of human hematopoiesis, infused with Nalm6 Luciferase+ cells. At day 5, mice were injected with 3×10⁶ CAR T cells or NT cells, generated from splenocytes harvested from mice humanized with the same HSPC donor by lentiviral transduction of an anti-CD19CAR.BBz. (B) IVIS images of mice injected with CAR T cells or NT cells. (C-D) Representative flow cytometry plots of GFP+CD19+ tumor cells, GFP-CD19+ healthy B cells, CD33+ myeloid and CD3+ T cells in BM of animals from each treatment at euthanization. One representative mouse per group is shown. (E) Percentage of CD33+HLA-DR+CD14+/CD15+ monocytes cells, VEGFR2+ in CD33+ myeloid cells, HIF1α^{high}CD33+ cells and CD33+ HLA-DRlow/CD14+/CD15+ MDSC in BM of mice treated with CAR T or NT cells at euthanization. (F) Volcano plot of DEGs in myeloid cells purified from the BM of treated mice with CAR T cells compared with mice treated with NT cells. (G) Newborn NSG mice were sub-lethally irradiated, and injected intrahepatically with human PB-mobilized CD34+ cells and BM cells from secondary PDX recipient. After tumor engraftment, mice were injected with CAR T cells or NT cells generated from HSPC-humanized mice. (H) Representative flow cytometry plots of T cells, myeloid cells,CD123+CD19+ tumor cells, CD123-CD19+ B healthy cells in PB of a representative mouse at 2 months post transplantation. (I) Representative flow cytometry plots of CD3+ cells expressing PD1, TIGIT, TGFBR2 in BM of a representative mouse treated with CAR T cells at euthanization.
**Supplemental** **Figure 5****.** Remodeling of BM microenvironment after CAR T infusion in tumor-bearing humanized mice. (A) Bioluminescence intensity (total flux per second) over time in mice receiving the indicated treatment. Two-way ANOVA with Turkey multiple comparison (*compared with NT). (B-C) Representative flow cytometry dot plot of monocytes, VEGFR2+CD33+ cells, HIF-1αhighCD33+ cells and MDSC in BM of mice treated with CAR T or NT cells. One representative mouse is shown. (D) Significantly-enriched KEGG (left) and Reactome (right) gene sets in purified myeloid cells of mice treated with CAR T or NT cells, FDR (-logpvaladj) < 0.05.

### EXAMPLES

### EXAMPLE 1

We hypothesized that the TME reacts to CAR T-cell mediated inflammation by accumulation of immune suppressor cells and induction of inhibitory pathways and molecules, leading to decreased treatment efficacy. To elucidate the involved cellular and molecular mediators, we performed scRNA-seq of BM samples from B-ALL patients collected at early time points (1-2 months) after treatment. CD45+CD3+ T cells, and CD45+/lowCD3-, encompassing the hematopoietic immune niche of the TME, were sorted and compared to cells of the corresponding patient's BM before CAR T-cell treatment at the moment of relapse. In parallel, manufactured CAR T-cell infusion products (IP) were sorted according to surface CAR expression (Figure 1A-B).

Eighteen sequencing libraries were analyzed from three B-ALL patients (Supplementary Table 1), for a total of 71.407 high quality cells at an average of 65.000 reads per cell. One of these patients had been treated with anti-CD19 CAR T cells engineered with the non-viral Sleeping Beauty transposon vector from donor T cells, referred to as CARCIK-CD19(5) ("investigational"), and two of them with commercial CAR T cells. Unbiased clustering was performed and 8 cell clusters were identified as CD4, CD8, NK, NKT, myeloid, B, plasmacytoid DC (pDC) and erythrocytes based on relative expression levels of lineage-specific genes (Figure 21). (6) CAR gene expression was detected in all post-infusion samples. We applied high-resolution clustering of CD3+ cells, to recognize a qualitatively enriched cluster in CAR+ cells and annotate it as putative CAR+ (Figure 1D). The identified 11 subpopulations were visualized using UMAP (Figure 1E).

We observed profound changes in the composition of the BM compartment in samples post CAR T-cell infusion compared to pre-infusion samples (Figure 1F). Remarkably, a complete disappearance of the B-cell associated cluster was observed after treatment, which correlates with the clinical data of the selected patients who achieved complete remission. Concomitantly, we noticed a relative increase in the fraction of myeloid cells post-treatment.

### EXAMPLE 2

We evaluated the abundance of the 6 transcriptionally distinct cell clusters identified in the BM-derived CD45+/lowCD3- cell compartments comparing samples post-infusion to pre-infusion (Figure 2A-B and SFigure 2A-B). In the myeloid cluster, Gene Set Enrichment Analysis (GSEA) across individual cell types using the Hallmark gene set collection(7) showed a significant enrichment of gene expression associated with IFN-α and IFN-γ response, IL-6/JAK/STAT3 signaling, inflammatory response, and hypoxia, associated with upregulation of HIF1α and its target gene, the angiogenic factor *VEGFA* (Figure 2D). Using metabolically scoped gene set collections manually curated from KEGG and Reactome databases,(8) we observed increased lipid metabolism, steroid biosynthesis, and glycosaminoglycan biosynthesis (SFigure 2C-D), which are associated with immunosuppressive myeloid cells.(9) Differential gene expression analysis revealed up-regulation of molecules that orchestrate myeloid-derived suppressor cell (MDSC) immunosuppressive functions, such as *TGFB1*, *ARG2*, *NOS1AP*, *ENTPD1* (CD39), *S100A9* and *S100A12* (Figure 2D). Moreover, *CCL2* and *TLR2,* which play a key role in MDSC migration,(10) and DC dysfunction,(11) respectively, were also up-regulated. We applied a specific signature for murine MDSCs by mapping the overexpressed genes to human orthologues using BioMart tools,(12) and found that myeloid cells from samples post-treatment display a higher resemblance to MDSCs (Figure 2E).

To validate and refine our findings, we expanded our study with a cohort of additional 20 matched BM samples (Supplementary Table 1) pre- and post-CAR T-cell treatment acquired using spectral flow cytometry. Identification of NK and monocytes was based on reference guidelines by the EuroFlow Consortium using the Infinicyt software. In line with our findings, we observed a significant increase in monocytes (median 3.7%, SD 5.8%, pre vs post 9.8%, SD 9.8%), NK (1.8%, SD 2.6% vs 3.7%, SD 3.6 %), and MDSCs (1.7%, SD 2.9%, vs 3.4%, SD 3.4%), complemented by a decrease in CD45low leukemic cells post-treatment (Figure 2F). To characterize specific treatment-related alterations in the myeloid compartment, we used manual gating. We found a significant increase of monocytes expressing high levels of CCR2, the receptor for CCL2, and TLR2 in total nucleated cells post-treatment, consistent with the scRNAseq data (Figure 2G). In sum, these data provide strong evidence for activation of signaling pathways in the TME that drive a skewing towards acquisition of an MDSC phenotype and immunosuppressive functions after CAR T-cell treatment.

### EXAMPLE 3

Having shown a remodeling of the myeloid compartment after CAR T-cell infusion, we explored changes occurring in the CD3+ component to identify subsets of T lymphocytes associated with the therapy which may affect the therapeutic efficacy. We applied dimensionality reduction to the CD45+CD3+ scRNA-seq data using UMAP (Figure 3A) and utilized Garnett classification to generate a final annotation.(13) We identified 10 distinct clusters, including CD4 naïve-like, Th1/Th17, Th2, IL-10 producing Type 1 regulatory T cells (Tr1), Treg, CD8 effector, early memory, exhausted, resident memory, and NKT cells (Figure 3B). Global cell type enrichment analysis using SingleR transcription-based cell assignments revealed an increase in NKT, Tr1, and exhausted CD8+ T cells post CAR T-cell infusion (Figure 3C). A higher expression of genes associated with cell cycle in endogenous T cells, as well as with IFN-α and IFN-γ response and TGF-β signaling in CAR T cells was observed. Interestingly, we found widespread inhibition of glycolysis and oxidative phosphorylation (OXPHOS), which have been previously associated with metabolic insufficiency exhibited by exhausted T cells (Figure 3D).(14) A shared signature of genes associated with lipid metabolism suggests that T cells sense a lipid-enriched and nutrient-deprived TME which have been reported to facilitate T-cell exhaustion (Supplementary Figure 2A-B).(15) Endogenous exhausted T cells showed maintenance of some resident memory features,(16, 17) with a higher expression of *TGFB1*, *IL7R,* and *IL-32,* while CAR T cells up-regulated expression of *HIF1A*, *TOX, EOMES,* and *TGFB1* (Figure 3E). Interestingly, *TCF7,* which is critical in driving differentiation of progenitor exhausted cells,(18) was up-regulated in CAR T cells as well as endogenous exhausted T cells.

To validate this finding at a cellular and protein level, we analyzed the lymphoid cells from the flow cytometry data using the Infinicyt software and showed that the number of CD3+ and CD3+CD8+ cells significantly increased while CD3+CD4+ cells decreased after treatment. This was also evident when we performed hierarchical consensus clustering using FlowSOM (Figure 3F-G). We analyzed the expression of the exhaustion markers by manual gating and observed an increase in the % of CD3+PD1+ (median 7.50%, SD 7.7%, pre vs post 16.5%, SD 11.9%), CD3+TIGIT+ (4.9%, SD 7.3% vs 12.6%, SD 10%) and CD3+TIM3+ (0.7%, SD 2.4% vs 6.2%, SD 11.2%) as well as double-positive TIGIT+PD1+CD3+ T cells (Figure 3H). Notably, CAR T cells showed increased expression of TIGIT, PD1 and TIM3. PD1 expression was also increased in resident CD4+ cells post-infusion (Figure 3I). Together, these data suggest that CAR T-cell treatment triggers remodeling of the BM TME, associated with acquisition of widespread T-cell exhaustion, affecting both CAR T cells and endogenous T cells, revealing generalized immunosuppression in the TME in response to CAR T-cell mediated bystander activation of endogenous immunity, as in chronic inflammation.(19, 20)

### EXAMPLE 4

Given our hypothesis that the interplay between anti-CD19 CAR T cells and the immune compartment of the TME affects long-term therapeutic efficacy in B-ALL, we assessed the role of TME factors on the duration of response (DOR) and the event-free survival (EFS) in the cohort of patients analyzed by flow cytometry (Supplementary Table 2). Specifically, we analyzed the frequency of MDSCs, CCR2+ monocytes, T cells and CAR T cells expressing PD1, and TIGIT post treatment. We found that a high % of CCR2 expression in monocytes after CAR T-cell treatment was only weakly associated with an increased DOR (hazard ratio (HR), 0.96, 95% CI, 0.92-1.00, p=0.05) but the association was stronger on EFS (HR, 0.95, 95% CI, 0.91-0.99, p=0.02). Conversely, higher % of CD3+PD1+ cells in endogenous T cells were significantly associated with decreased DOR (HR, 1.05, 95% CI, 1.01-1.10, p=0.03) and EFS after CAR T-cell treatment (HR, 1.05, 95% CI, 1.01-1.10, p=0.03, Supplementary Table 2). The median survival was 1.13 years (0.24-3.46). Therefore the acquisition of exhaustion in endogenous T cells after CAR T-cell infusion appears to be clinically relevant in our cohort, as we find that an increased proportion of PD1+ T cells has a higher risk of shorter DOR and EFS. Moreover, increased proportion of PD1+ T cells can be used as a biomarker to predict poor response to CAR T-cell therapy and stratify the patients potentially benefitting of combinatorial therapy. Our finding also highlight the importance of incorporating combinatorial strategies that dampen excessive inflammation in the TME, such as targeting IFN, to prevent CAR T-cell exhaustion and promote beneficial rather than detrimental endogenous T-cell recruitment.

### EXAMPLE 5

To unravel the mechanisms leading to the T-cell dysfunction associated with decreased long-term therapeutic efficacy, we explored the intercellular communication between different cell compartments, applying the computational method NicheNet to our scRNA-seq data.(21) By using all cellular components of the niche as sender, and myeloid cells as receiver, we found CAR T cells and endogenous T cells to contribute to induction of HIF1α, *STAT1* and *IL6R* expression in myeloid cells (Figure 4A). Notably, TGF-β and IFN-γ expressed by T cells were predicted to be part of the network of communication signals mediating this effect (Supplementary Figure 3A). We then used NicheNet to predict signaling pathways from the niche influencing CAR T-cell fate and behavior. We observed induction of *NR4A2,* and *IRF1*, previously associated with T-cell exhaustion,(22, 23) *TGFB1*, the BM-attractant *CXCR4, SLC7A5* transmembrane amino acid transporters, and *ITGA4* (Figure 4B and Supplementary Figure 3B). *TGFB1*, *SLC7A5,* and *ITGA4* were also induced by the BM niche in endogenous exhausted T cells, in parallel to *TGFBR2, and NFKB* (Supplementary Figure 3C). Interestingly, myeloid cells were the main driver for the generation of an autocrine TGF-β signaling in both CAR T cells and endogenous T cells (Supplementary Figure 3B-D). Consistent with DEG analysis in the myeloid cluster, VEGFA, VEGFB and CCL2 were again identified as key players. We detected a slight increase of phospho-smad signal post-treatment (Supplementary Figure 3E), associated with Smad2 and Smad3 upregulation (Supplementary Figure 3F). The generalized increase in HIF1α, *TGFB1*, and *TGFBR2* expression (Figure 4C) was validated by digital droplet (dd)-PCR in purified CD3+ and CD3- BM cell subsets (Supplementary Figure 4A).

To determine whether hypoxia drives CAR T-cell exhaustion upon chronic T-cell activation, anti-CD19 CAR T cells were repeatedly stimulated with target cells, consisting of the B-ALL Nalm6 cell line, in normoxic (20% O₂) and hypoxic conditions (1.5% O₂, Figure 4D). Short-term co-culture in hypoxic conditions did not affect CAR T-cell functionality (Figure 4E). However, decreased proliferation was observed upon persistent antigen exposure under hypoxic conditions, resulting in decreased % of CAR T cells (Figure 4F and Supplementary Figure 4B), increased expression of TGFBR2 (Supplementary Figure 4C) and a higher proportion of terminal effector CD3+CAR+ cells (Supplementary Figure 4D). Further, we observed decreased CAR T-cell functionality associated with incomplete clearance of target cells at the end of the assay (Figure 5G). Therefore, we assessed whether inhibition of HIF1α or blockade of the VEGF-A/VEGFR2 interaction prevents T-cell dysfunction. CAR T-cell proliferation, together with target cell clearance, was increased by anti-VEGFR2 blocking antibodies (Ramucirumab) and, to some extent, by pharmacological inhibition of HIF1α with PX478 during persistent antigen exposure under hypoxic conditions (Figure 4H). Thus, CAR T-cell mediated inflammation favors a hypoxic microenvironment that, during persistent stimulation, drives T cells towards a dysfunctional state that may antagonize the effects of CAR T-cell therapy. Importantly, inhibition of either VEGFR2 or HIF1α reinvigorated CAR T cells and restored their anti-tumor functions. Therefore, combination of CD19 CAR T cell therapy and inhibition of either VEGFR2 or HIF1α may be exploited in the clinic to enhance the efficacy of CAR T-cell treatment in the hypoxic TME.

### EXAMPLE 6

To validate our findings in vivo, we reconstituted newborn NSG mice by intrahepatic injection of CD34+ HSPCs and, after establishment of human hematopoiesis, transplanted with Nalm6, modified to express luciferase (Figure 5A). To avoid xenograft rejection, n-hu-Nalm-NSG mice received 3 × 10⁶ anti-CD19 CAR T cells generated from the splenocytes harvested from mice humanized with the same HSPC donor. CAR T cells mediated robust anti-leukemic activity in vivo, as demonstrated by the flux signal, compared to mice treated with NT cells (Figure 5B and Supplementary Figure 5A). At the time of euthanization, mice treated with CAR T cells showed almost complete clearance of leukemic blasts in the BM, but healthy B-cell reconstitution, associated with absence of human CD3+ T-cell engraftment (Figure 5C-D). Notably, we observed an increased frequency of human monocytes in the BM of CAR T cell-treated mice compared with control mice, associated with increased percentage of VEGFR2+CD33+ cells (median 5.2%, SD 2.8% in CAR T vs NT 0.9%, SD 0.6%) and HIF-1α^{high}CD33+ cells (6.9 %, SD 0.5% vs 1.3%, SD 1.9 %), as well as MDSCs (0.8%, SD 0.5% vs 0.3%, SD 0%, Figure 5E and Supplementary Figure 5B-C). Purified myeloid cells were analyzed with bulk transcriptomics and confirmed our abovementioned scRNA-seq data, including increased expression of CCL2 and genes involved in biological oxidation and steroid biosynthesis (*CYP26B1, CYP27C1*), ROS *(SLAMF8, PXDNL, MAOA),* and MDSCs (*MMP19, NOS1AP*) (Figure 5F and Supplementary Figure 5D).

Since T cells were no longer detected after tumor clearance in the n-hu-Nalm-NSG model, we developed combined HSPC-humanized / PDX mice, by concomitant intrahepatic injection of CD34+ HSPCs and BM cells from secondary PDX recipient (Figure 5G). Remarkably, our n-hu-PDX-NSG model recapitulates the human tumor immune microenvironment, reconstituting a functional human immune system, including CD3+ T cells, CD19+ B cells and CD33+ myeloid cells, as well as the human CD19+CD123+ tumor (Figure 5H). In this model, at sacrifice all treated mice showed substantial leukemia infiltration in the BM with absence of other cell compartments, except for CD3+ cells in CAR T cell-treated mice. As seen in the patient samples, T cells showed high expression of TIGIT (median 79.4% of TIGIT+CD3+ cells, SD 13.4%), PD1 (47.1%, SD 18.1%), and TGFBR2 (55.8%, SD 17.1%, Figure 5I).

Overall, our mouse data provide experimental confirmation of the remodeling of the BM immune niche after CAR T-cell treatment, characterized by increased hypoxia, leading to skewing of the myeloid compartment towards MDSCs, and increased numbers of exhausted T cells and showing accumulation of VEGFR+ and HIF1αhigh myeloid cells, MDSCs and acquired expression of PD1, TIGIT, and TGFBR2 on T cells after CAR T-cell infusion

Overall, our data suggest that CAR T cells promote myeloid, and MDSC expansion in the TME in patients with B-ALL, ultimately leading to T-cell exhaustion as an active feedback loop in response to CAR T-cell-mediated inflammation. Mechanistically, the acquisition of an exhausted phenotype in endogenous T cells and CAR T cells is induced by the coordination of chronic inflammation and microenvironmental stressors such as IFN response, VEGF and HIF1α signaling, and may antagonize the effect of the therapy. Pharmacological inhibition of VEGFR2 and HIF1α counteract CAR T-cell dysfunction, setting the way for combinatorial treatments with the aim to increase CAR T-cell functions in the TME.

**Supplemental Table 1: Patients' characteristics**

| Patient ID | Age (years) | Sex | Genetic abnormality | Clinical Study - Infusion product | Respon se | Analyses |
|---|---|---|---|---|---|---|
| #001 | 2 | F | t(4,11) | FT01 CARCIK-CD19 | CR | Hematology and scRNAseq |
| #002 | 17 | F | Negative for all translocations | Commercial anti-CD19 CAR T | CR | Hematology and scRNAseq |
| #003 | 6 | F | Negative for all translocations | Commercial anti-CD19 CAR T | CR | Hematology and scRNAseq |
| #004 | 5 | F | t(9;18) | FT01 CARCIK-CD19 | NR | Hematology |
| #005 | 27 | M | hyperdiploid | FT01 CARCIK-CD19 | NR | Hematology |
| #006 | 56 | M | Negative for all translocations | FT01 CARCIK-CD19 | NR | Hematology |
| #007 | 62 | F | t(9;22) Bcr/Abl1 | FT01 CARCIK-CD19 | CR | Hematology |
| #008 | 45 | F | t(9;22) Bcr/Abl1 | FT01 CARCIK-CD19 | CR | Hematology |
| #009 | 10 | M | t(4;11) | FT01 CARCIK-CD19 | NR | Hematology |
| #010 | 7 | M | Complex karyotype | FT01 CARCIK-CD19 | CR | Hematology |
| #011 | 32 | F | Negative for all translocations | FT01 CARCIK-CD19 | NR | Hematology |
| #012 | 39 | F | Negative for all translocations | FT01 CARCIK-CD19 | CR | Hematology |
| #013 | 63 | M | t(9;22) Bcr/Abl1 | FT01 CARCIK-CD19 | CR | Hematology |
| #014 | 52 | F | Hyperdiploid | FT01 CARCIK-CD19 | CR | Hematology |
| #015 | 30 | M | Complex karyotype | FT01 CARCIK-CD19 | CR | Hematology |
| #016 | 28 | M | t(9;22) Bcr/Abl1 | FT01 CARCIK-CD19 | NR | Hematology |
| #017 | 35 | F | Not available | FT01 CARCIK-CD19 | NR | Hematology |
| #018 | 60 | F | t(9;22) Bcr/Abl1 | FT01 CARCIK-CD19 | CR | Hematology |
| #019 | 46 | M | t(9;22) Bcr/Abl1, p190, mut T315 I | FT01 CARCIK-CD19 | NR | Hematology |
| #020 | 36 | M | t(9;22) Bcr/Abl1 | FT01 CARCIK-CD19 | CR | Hematology |
| #021 | 37 | M | t(9;22) Bcr/Abl1 | FT01 CARCIK-CD19 | CR | Hematology |
| #022 | 38 | F | t(9;22) Bcr/Abl1 | FT01 CARCIK-CD19 | CR | Hematology |
| #023 | 26 | F | Negative for all translocations | FT01 CARCIK-CD19 | CR | Hematology |
| #024 | 4 | M | Ph-like | Commercial anti-CD19 CAR T | CR | Hematology |
| #025 | 13 | F | t(1;19) | Commercial anti-CD19 CAR T | CR | Hematology |
| #026 | 21 | M | Negative for all translocations | Commercial anti-CD19 CAR T | PR | Hematology |
| #027 | 0 | F | t(4;11) | Phase 1/2 anti-CD 19 CAR T | CR | Hematology |
| #028 | 7 | M | Complex karyotype | Commercial anti-CD19 CAR T | CR | Hematology |
| #029 | 2 | M | t(11;19) | Commercial anti-CD19 CAR T | CR | Hematology |
| #030 | 1 | M | Negative for all translocations | Phase 1/2 anti-CD 19 CAR T | CR | Hematology and flow cytometry |
| #031 | 4 | M | t(11;19) | Phase 1/2 anti-CD 19 CAR T | CR | Hematology and flow cytometry |
| #032 | 8 | M | t(9;22) Bcr/Abl1 | Commercial anti-CD19 CAR T | CR | Hematology and flow cytometry |
| #033 | 10 | M | t(12;21) | Commercial anti-CD19 CAR T | CR | Hematology and flow cytometry |
| #034 | 4 | F | t(4;11) | Commercial anti-CD19 CAR T | CR | Hematology and flow cytometry |
| #035 | 8 | M | Negative for all translocations | Commercial anti-CD19 CAR T | NR | Hematology and flow cytometry |
| #036 | 1 | F | KMT2A rearrangement | Commercial anti-CD19 CAR T | CR | flow cytometry |
| #037 | 19 | F | Negative for all translocations | Commercial anti-CD19 CAR T | CR | flow cytometry |
| #038 | 11 | F | t(9;22) Bcr/Abl1 | Commercial anti-CD19 CAR T | CR | flow cytometry |
| #040 | 4 | M | t(9;22) Bcr/Abl1 | Commercial anti-CD19 CAR T | CR | flow cytometry |
| #040 | 8 | M | Complex karyotype | FT03 CARCIK-CD19 | CR | flow cytometry |
| #041 | 16 | F | Negative for all translocations | FT03 CARCIK-CD19 | CR | flow cytometry |
| #042 | 47 | F | t(9;22) Bcr/Abl1 | FT03 CARCIK-CD19 | CR | flow cytometry |
| #043 | 45 | F | t(9;22) Bcr/Abl1 | FT03 CARCIK-CD19 | CR | flow cytometry |
| #044 | 66 | M | Negative for all translocations | FT03 CARCIK-CD19 | CR | flow cytometry |
| #045 | 48 | M | Ph-; trisomy 8 monosomy 7 | FT03 CARCIK-CD19 | CR | flow cytometry |
| #046 | 42 | F | Negative for all translocations | FT03 CARCIK-CD19 | CR | flow cytometry |
| #047 | 58 | M | J beta 1.1 | FT03 CARCIK-CD19 | CR | flow cytometry |
| #048 | 59 | M | t(9;22) Bcr/Abl1 | FT03 CARCIK-CD19 | CR | flow cytometry |
| #049 | 49 | F | Mixed phenotype | FT03 CARCIK-CD19 | CR | flow cytometry |

Abbreviations: F, female; M, male; CR, complete response; NR, no response; scRNAseq, single-cell RNA sequencing

**Supplemental Table 2: EFS and DOR of the patients analyzed by spectral flow cytometry**

| **Patient ID** | **DOR (years)** | **Event** | **EFS (years)** | **Event** |
|---|---|---|---|---|
| #035 | - | - | 0.08 | 1 |
| #040 | 0.07 | 1 | 0.15 | 1 |
| #047 | 0.18 | 1 | 0.27 | 1 |
| #044 | 0.23 | 1 | 0.31 | 1 |
| #049 | 0.24 | 1 | 0.31 | 1 |
| #039 | 0.27 | 0 | 0.34 | 0 |
| #043 | 0.33 | 1 | 0.41 | 1 |
| #033 | 0.45 | 1 | 0.52 | 1 |
| #041 | 0.48 | 1 | 0.56 | 1 |
| #037 | 0.49 | 1 | 0.57 | 1 |
| #048 | 0.51 | 0 | 0.59 | 0 |
| #038 | 0.51 | 0 | 0.58 | 0 |
| #046 | 0.67 | 1 | 0.72 | 1 |
| #036 | 0.71 | 0 | 0.79 | 0 |
| #031 | 0.81 | 1 | 0.89 | 1 |
| #045 | 1.05 | 0 | 1.13 | 0 |
| #032 | 1.13 | 1 | 1.21 | 1 |
| #042 | 1.46 | 0 | 1.54 | 0 |
| #030 | 1.48 | 0 | 1.55 | 0 |
| #034 | 3.26 | 0 | 3.34 | 0 |

Abbreviations: DOR, duration of response; EFS, event-free survival

### MATERIAL AND METHODS

The material and methods of the above examples are described in the following paragraphs.

### Patient Samples

Data in this study were generated from patients enrolled in the FT01CARCIK Phase I/IIb clinical trial (NCT03389035), in the FT03CARCIK Phase II study (NCT05252403) or from autologous CAR T cells in the context of either phase I/II trial or commercial cell therapy (tisagenlecleucel). All patients or their legal guardians provided written informed consent for sample collection and analysis. Studies were in accordance with the declaration of Helsinki and Ethical approval was obtained. Patients underwent CAR T treatment at Fondazione Monza e Brianza per il Bambino e la sua Mamma (MBBM), Monza, Italy (pediatric patients) or at Azienda Socio Sanitaria Territoriale Papa Giovanni XXIII, Bergamo, Italy (adult patients).

### Frozen BM Preparation, Flow Cytometry, and Cell Sorting

For scRNA-seq analysis, samples consisted of frozen BM samples. Cell counts and viability after sorting were optimal and sufficient to proceed with the scRNA-seq analysis. Frozen human BM and IP cryotubes were rapidly thawed and stained with hCD45 PO (clone HI30, Invitrogen) and CD3 PerCP (clone SK7, BD) or with CD19 biotin protein (Miltenyi) followed by Biotin APC (REA746, Miltenyi) and CD3 PercP (clone SK7, BD) antibodies, respectively. BM cells were separated into CD45+CD3+ and CD45+CD3- fractions, whereas IP was separated into CD3+CAR+ and CD3+CAR- cells by flow cytometry-based sorting (BD FACS ARIA III analyser). All samples were gated based on physical parameters, followed by the exclusion of doublets and death cells (DAPI high). After sorting, cells were counted and checked for viability.

### scRNA-seq

The scRNA-seq libraries were generated using Chromium Single Cell 5 Reagent Kit of the 10x Genomics (v3.1). Non-PCR duplicates with valid barcodes and Unique molecular identifiers (UMIs) were employed to create the gene-barcode matrix according to standard pipelines from the Cell Ranger software (Chromium Single Cell Software Suite). To enable identification of cells expressing the anti-CD19 CAR transcripts, sequences corresponding to the recombinant mouse CDR3 region directed against CD19 molecule and the leader were added to the reference genome and annotated. Individual libraries were then analyzed using the Scanpy package 9 and putative doublets were removed using the scrublet package with the threshold set to 0.2 10,11. To limit technical artifacts, libraries were then downsampled to have average counts per cell values comparable to the least represented library (-2500 counts per cell on average). All libraries were subsequently concatenated into a single dataset. Genes expressed in less than 1/1000 cells (i.e., 72 cells) were removed as low expressed genes. To exclude low quality cells, barcodes with less than 200 genes were removed. Furthermore, cells deviating for more than a fixed number of mean absolute difference (MAD) from the median of selected metrics were excluded from the analysis. The metrics evaluated percentage of mitochondrial transcripts (nMAD=4, threshold ∼ 20%), percentage of ribosomal transcripts (nMAD=2, threshold ∼ 50%), number of UMI (nMAD=3, threshold ∼ 6000), number of expressed genes (nMAD=3, threshold ∼ 2700). In the end 71,407 cells were retained for further analysis. The data were normalized to a total expression of 10⁴ counts per cell and log transformed. Highly variable genes (HVG) were selected using the corresponding function from the scanpy package, increasing the min_mean parameter to 0.1 which yielded 909 HVG. For visualizing the data, the dimensionality of the matrix was reduced further to project the cells in two-dimensional space using PCA followed by UMAP (considering 25 PCs, 30 nearest neighbours, min_dist=0.1). To reduce the effect of technical artifacts onto cell embeddings, the HARMONY algorithm12 was applied to the dataset before UMAP projection to achieve better integration.

### Cell type annotation

For the CD3- compartment, clusters were assigned to identified cell types based on the expression of defined population markers using unsupervised clustering (6). For the classification of cells from the CD45+/CD3+ compartments, we relied on a previously described strategy employing a hierarchical logistic classifier(13). Briefly, at each level of the cellular hierarchy, a set of positive and negative markers were selected to identify populations of interest (marker file) and were used to select high-confidence cells from a reference dataset (GSE161529). Residual cells not assigned to any of the target classes were further analyzed and manually annotated according to differentially expressed gene markers. For MDSC classification, the signature transcriptional activity was evaluated at single cell level by taking the singular value decomposition (SVD) score on the signature-restricted gene expression matrix.

### Differential abundance evaluation

To evaluate differential cell-type abundance across time points in CD3- cells, libraries of interest were subset and retained from the full dataset and both PCA and UMAP representation were computed. MILO algorithm was then employed to highlight cellular populations with differential representation across conditions (design= ~0+timepoint, contrast= timepointT1-timepointT0). To identify neighborhoods we considered k= 30 nearest neighbours over 30 PCs and set the attribute prop= 0.1 while all other parameters were left as default.

Due to the higher transcriptional homogeneity observed in CD3+ data subset, we evaluated differential abundance considering the overall relative proportion of a specific cell subtype across time points.

### Cellular crosstalk evaluation

To predict intracellular communication, we used NicheNet, a computational tool for predicting ligand-receptor binding and target genes among interacting cells by combining their expression data with prior knowledge of signaling pathways and gene regulatory networks 2. Of interest, the recently added 'MultiNicheNet' procedure allows to compare signaling within a specific niche across different condition (time points in our case). We defined different niches in which, for each iteration, we focused our analysis on a specific cell type as receiver (myeloid, CD8_exhausted, and CAR T cells respectively) while leaving all other cell types as possible senders. This approach highlights ligand-receptor pairs, which showed differential expression across conditions and were likely to induce the expression of observed DEG target for the receiver population. MultiNicheNet analysis was performed setting expression_pct = 0.10, specificity_score_LR_pairs = "min_lfc", include_spatial_info_sender and include_spatial_info_receiver both set to FALSE, lfc_cutoff = 0.15, specificity_score_targets = "min_lfc", top_n_target = 250. Relative weights for prioritization were set at scaled_ligand_score= 4, scaled_ligand_expression_scaled= 1, ligand_fraction= 1, scaled_ligand_score_spatial= 0, scaled_receptor_score= 0.5, scaled_receptor_expression_scaled= 0.5, receptor_fraction= 1, ligand_scaled_receptor_expression_fraction= 1, scaled_receptor_score_spatial= 0, scaled_activity= 0, scaled_activity_normalized= 1, bona_fide= 1

### Phenotypical Analysis

To identify molecular pathways altered within cell types at different time points, we performed a GSEA analysis on DEG for each cell type (as returned by calling rank_genes_groups from the scanpy package onto a specific cell type grouped by time points). To evaluate different aspects of cellular behavior, we tested the Hallmark collection from the Molecular Signatures Database (MSigDB 15) by GSEA as well as the hand-refined and metabolically scoped KEGG and Reactome collections described in scMetabolism 3.

### CAR T-cell detection

For the identification of CAR+ cells in the final dataset, BM CD45+CD3+ libraries were preliminarily explored individually. Cells expressing the annotated CAR sequences were directly tagged as 'cart' cell. Following data clustering using unsupervised community detection algorithms (leiden algorithm 17), the 'cart' label was then extended to clusters enriched in CAR-expressing cells.

### Flow Cytometry Validation

BM samples from treated patients were thawed and stained with a 30-colors panel. Data were acquired on a Cytek Aurora (Cytek^{®} Biosciences) and analyses were performed using Infinicyt (Cytognos), DIVA software, and FlowSOM.

### Unsupervised flow-cytometric analysis with FlowSOM

All fluorescence conjugated antibodies are listed in Supplemental Table S3 and Supplemental Table S4. We used the raw fcs files from 36 paired samples (pre and +1 month) from 18 patients as described above. First, we manually gated on live cells, according to physical parameters and CD45 expression, and applied a spillover matrix using polygonGate and compensation functions of flowCore package (v.2.12.0). We performed logicle transformation employing FCSTransTransform function. The quality of transformed fcs files was assessed using PeacoQC function of PeacoQC package (v 1.11.0). Processed fcs were aggregated together in a 2×10⁶ events file (roughly 55000 live cells per file). Clustering analysis was performed with FlowSOM R package (v2.8.0). The first clustering step was performed using a 15x15 grid producing 225 clusters and 10 metaclusters to identify Monocytes and Lymphocytes and to eliminate the remaining debris; we employed the same markers used in the manual gating strategy. A second level of clustering was performed on Monocytes (6x6 grid and 15 metaclusters) and Lymphocytes (15x15 grid and 15 metaclusters) to dissect their composition. For each level, counts, percentages and MFI were extracted from each subset using GetFeatures function of FlowSOM package. Downstream statistical analysis was performed using GraphPad Prism (version 10). UMAP was computed using the umap function from the uwot R package (v0.1.16.9) and the n_neighbors parameter set to 15.

### Analysis of TGF-β signaling

Activation of the TGF-β signaling was verified through phospho-flow analysis of the Smad proteins (basal phosphorylation). To evaluate the phosphorylation of SMAD proteins, thawed BM cells were rested in RPMI medium containing 0.1% FBS for 2 hours at 37°C. Cells were then fixed with 1.5% paraformaldehyde and permeabilized with 90% ice-cold methanol prior to staining with anti-Smad2 (pS465/pS467)/Smad3 (pS423/pS425) antibody (clone O72-670,BD Biosciences) or isotype matched IgG, anti-CD7 ECD (Beckman Coulter) and anti-CD45 PerCP (BD).

### ddPCR analysis

BM cells were separated into CD45+CD3+ and CD45+CD3- fractions through CD3+ positive selection on LS column with CD3 MicroBeads (Miltenyi). RNA was extracted with miRNeasy micro kit (QIAGEN). 10 ng of RNA was retrotranscribed with SuperScript^{™} IV Reverse Transcriptase (Thermo Fischer) and Digital Droplet ^{™} PCR (ddPCR^{™}) (BIO RAD) was performed for the evaluation of HIF1α (dHsaCPE5033624), TGFB1 (dHsaCPE5055188), VEGFA (dHsaCPE5034754) genes. Analyses were performed with QuantaSoft Analysis pro Software ^{™} (BIORAD).

### In vitro Co-culture

anti-CD19 CAR T cells were manufacture as previously described in (5, 24). T cells were obtained from anonymized healthy donors from the blood donation service. CAR T cells were cultured with Nalm-6 for 24 hours (acute stimulation) at effector to target (E:T) ratios of 1:1 and 1:5 under normoxia (20% O₂) or hypoxia (1.5% O₂) compared to NT cells. Cytotoxicity was calculated as 100-(% live Nalm6 in co-culture with effector cells /% live Nalm6 alone) ×100. To model persistent antigen exposure, CAR T cells were cultured with Nalm6 at a E:T ratio of 1:1 and restimulated with target cells at day 3 and day 6. All conditions were run in triplicates. For the drug treatment, CAR T cells were cultured with Nalm-6 at a E:T ratio of 1:3 under hypoxia (1% O2) in the presence of anti-VEGFR2 antibody (ramucirumab, Selleck Chemicals) or PX478 (CAT #S7612, Selleck Chemicals) at 10µM and 50 µg/ml, respectively, and re-stimulated with target cells at day 3 and day 6. All conditions were run with two donors in duplicates and anti-VEGFR2 antibody and PX478 were replenished at the same concentration every 36 hours or every re-stimulation, respectively.

Cells were stained with a 18-colors panel. All fluorescence conjugated antibodies are listed in Supplemental Table S5. Data were acquired on a Cytek Aurora (Cytek^{®} Biosciences) and flow cytometric analyses were performed using Infinicyt (Cytognos) and FlowJo.

### In vivo studies

NSG (NOD.Cg-Prkdcscid Il2rgtm1Wjl/SzJ) mice were humanized as previously reported.(24) n-hu-Nalm-NSG mice were generated by transplanting Nalm6 after the establishment of the human hematopoiesis.(25) n-hu-PDX-NSG mice were generated by sub-lethal irradiation of newborn mice and co-transplantation of healthy CD34+ HSPCs from mobilized peripheral blood cells and patient-derived xenograft cells (Patient ID #003).(26) Animal experiments were conducted in compliance with procedures approved by the Veterinäramt des Kantons Zürich, Switzerland (194/2018 and 121/2022) and by the Italian Ministry of Health (approval no. 907/2016-PR). The animals were kept in the animal facilities of the University Hospital of Zürich and the Schlieren Campus of Zürich and of the Milano-Bicocca University. Splenocytes were harvested from mice humanized with CD34+ HSPCs from residual, left-over mobilized peripheral blood cells collected from stem cell infusion bags and processed by the biobank of the department of Medical Oncology and Hematology, University Hospital Zurich. The donor gave written consent. Studies were in accordance with the declaration of Helsinki and approved by the Cantonal Ethical Board Zurich, Switzerland (2019-01744). Anti-CD19 CAR T cells were produced by transduction with lentiviral vectors as previously described (24). The viral vector encoding the anti-CD19 CAR (clone FMC63), including the 41BB costimulatory domain, and the RQR8 sequences previously cloned in the pCDH-EF1α-MCS-T2A-GFP lentiviral plasmid was produced as previously reported (24). T-cell activation was performed with CD3/CD28 Dynabeads^{®} (Thermo Fisher Scientific) followed by transduction with LV in the presence of 8 µg/ml Polybrene (Santa Cruz Biotechnology, #134220) the following day. On day 3, beads were removed with magnets. LV CAR T cells were cultured for 7 days, purified with FITC-Micro Beads (Miltenyi) after incubation with hCD34 FITC (ThermoScientific) and purified cells were expanded for additional 5-7 days before cryopreservation or immediate use.

### Tumor-bearing HSPC-humanized mice

For the experiment depicted in Figure 6a, newborn pups were sub-lethally irradiated with 150 cGy with an RS-2000 irradiator (Rad Source, Buford, GA, USA) and transplanted by intrahepatic injection of 650.000-1×10⁶ CD34+ HSPCs from mobilized peripheral blood cells collected as described above. Levels of chimerism were monitored by flow cytometry of PB post-trans plantation and when the level demonstrated successful engraftment (i.e. >1%human CD45 positive cells in the peripheral blood), mice were infused with 0.5×10⁶ luciferase-GFP expressing Nalm-6. Animals with positive luciferase signal on day 5 were allocated to receive 3×10⁶ purified CAR+ T cells generated from splenocytes collected from HPCS-humanized mice with the same HSPC donor. For weekly bioluminescent imaging (BLI), mice were anesthetized and received 150 mg/kg bodyweight D-luciferin (PerkinElmer, Inc, Wal tham, MA, USA) as intraperitoneal injection. Image acquisition was performed on a Xenogen IVIS 200 machine (PerkinElmer) with the Living Image 149 Software. Animals were euthanized 21 days after T cell transfer. Hematological organs (PB, BM, and spleen) were collected and processed for flow cytometry analysis. The PDX model was established with BM material (Patient ID #003) as previously described (26). For the experiment reported in Figure 6g, newborn pups were sub-lethally irradiated with 150 cGy and co-transplanted by intrahepatic injection of 750.000 CD34+ HSPCs and 750.000 BM cells from the secondary leukemic recipients. After tumor engraftment, mice were treated with 3×10⁶ anti-CD19 CAR T cells generated from splenocytes from mice humanized with the same HSPC donor. Animals were euthanized 24 days after T-cell transfer. For flow cytometry analysis, cells from BM were stained with fluorescence conjugated antibodies listed in Supplemental Table S6.

HIF1α was detected in BM purified cells by intracytoplasmic staining (Fixation/Permeabilization Solution Kit, BD Bioscience) with anti-hHIF1α PE (clone546-16, Biolegend).

### RNA-seq

BM cells were thawed, negatively purified using anti PE-Micro Beads (Miltenyi) after incubation with anti-hCD19 and anti-mCD45 PE antibodies, and lysed directly with Qiazol (Qiagen). Total RNA was isolated and purified with miRNeasy micro Kit (Qiagen, Germany). For library preparation, we used Universal Plus Total RNA-Seq with NuQuant (Tecan Genomics). Analysis of RNA-seq data was performed using Galaxay Europe (https://doi.org/10.1093/nar.gkae410). Briefly, after quality check, transcripts were aligned using Bowtie2 (version 2.5.3). Subsequently, counts were extracted using FeatureCounts (Version 2.0.3) and differentially expressed genes were identified using Deseq2 (version 1.40.2). Gene set enrichment analysis was performed using Web based gene set analysis toolkit (https://www.webgestalt.org/).

### Statistics

Statistical analyses were performed in Graph Pad Prism Version 10 (GraphPad Software, Inc., La Jolla, CA) and SAS (v 9.4). Quantitative variables were described in terms of means ± stardard deviation (SD) and the degree of linear association was quantified by the Pearson correlation coefficient. Between groups comparisons were performed using the t-test when the groups were two and the ANOVA for three groups. Paired comparisons were performed by means of the Wilcoxon paired test. EFS was defined as the time from the date of CAR T-cell infusion to the earliest of the following events: no Complete Remission (CR), relapse or death from any cause, while DOR started from CR to relapse or death due to any cause, whichever occurred first. Patients were censored at the last follow-up in case no events occurred. To assess the impact of continuous variables on DOR and EFS we used the Cox model.

**Supplemental Table 3: Flow cytometry 30 color panel for validation**

| Fluorophore | Marker | Clone | Source |
|---|---|---|---|
| BUV395 | CCR7 | 2-L1-A | BD |
| BUV496 | CD45RA | 5H9 | BD |
| BV421 | CD84 | 2G7 | BD |
| V500 | CD45 | 2D1 | BD |
| BV605 | CD62L | DREG-256 (562719) | Biolegend |
| BV650 | CD27 | L128 | BD |
| BV711 | TIGIT | A15153G | Biolegend |
| BV750 | CD 127 | HIL-7R-M21 | BD |
| BV786 | CD3 | SK7 (563800) | BD |
| VB515 | CD19 CAR | REA746 | Miltenyi |
| BB700 | BTLA | 746166 | BD |
| PE | PD-1 | EH12.1 | BD |
| PE-CF594 | CD8 | RPA-T8 | BD |
| PECy7 | CD117 | 104D2 | BD |
| APC | TGFBR2 | W17055E | Biolegend |
| Alexa 647 | TIM3 | 7D3 | BD |
| APC-R700 | CD56 | NCAM16.2 (657886) | BD |
| APC-Cy7 | CD25 | M-A251 | Biolegend |
| APC/Fire 810 | CD4 | SK3 | BD |
| BUV661 | CD15 | W6D3 | BD |
| PB | HLA-DR | L243 | Biolegend |
| PEcy5 | CD33 | WM53 | Biolegend |
| BV570 | CD14 | M5E2 | Biolegend |
| BUV737 | TLR2 (CD282) | 11G7 | BD |
| BUV805 | CD11b | D12 | BD |
| BV480 | CD226 | 11A8 | BD |
| BUV615 | LAG3 (CD223) | T47-530 | BD |
| BUV 563 | CD16 | 3G8 | BD |
| Zombie NIR | Live cells | | Biolegend |

**Supplemental Table 4: Flow cytometry 7 color panel for validation**

| Fluorophore | Marker | Clone | Source |
|---|---|---|---|
| FITC | CD6 | BL-CD6 | Biolegend |
| BV786 | CD3 | SK7 (563800) | BD |
| V500 | CD45 | 2D1 | BD |
| PE-CF594 | CD8 | RPA-T8 | BD |
| APC-H7 | CD4 | SK3 | BD |
| BV605 | CCR2(CD192) | K036C2 | Biolegend |

**Supplemental Table 5: Flow cytometry 18 color panel for hypoxic co-colture validation**

| Fluorophore | Marker | Clone | Source |
|---|---|---|---|
| Zombie NIR | Live cells | | Biolegend |
| APC/Fire 810 | CD4 | SK3 | BD |
| BUV496 | CD45RA | 5H9 | BD |
| APC-Cy7 | CD25 | M-A251 | Biolegend |
| V500 | CD45 | 2D1 | BD |
| BV605 | CD62L | DREG-256 (562719) | Biolegend |
| BV650 | CD27 | L128 | BD |
| BV711 | TIGIT | A15153G | Biolegend |
| BV750 | CD 127 | HIL-7R-M21 | BD |
| BV786 | CD3 | SK7 (563800) | BD |
| VB515 | CD19 CAR | REA746 | Miltenyi |
| APC | CD10 TGFBR2 | 746166 | BD |
| PE | PD-1 | EH12.1 | BD |
| PE-CF594 | CD8 | RPA-T8 | BD |
| PECy7 | CD19 | HIB19 | BD |
| BV480 | CD226 | 11A8 | BD |
| BV615 | LAG3 | T47-530 | BD |
| Alexa 647 | TIM3 | 7D3 | BD |

**Supplemental Table 6: Flow cytometry antibodies list for in vivo staining**

| Fluorophore | Marker | Clone | Source |
|---|---|---|---|
| Zombie NIR | Live cells | | Biolegend |
| V450 (PB) | hCD45 | HI30 | Invitrogen |
| PerCP | mCD45 | 30-F11 | Biolegend |
| BV711 | CD33 | WM53 | Biolegend |
| PE | CD19 | SJ21C1 | eBiosciences |
| APC | CD3 | OKT3 | Biolegend |
| APC | TGFBRII | W17055E | Biolegend |
| FITC | CD19 | SJ21C1 | Biolegend |
| AF700 | CD8 | SK1 | Biolegend |
| BV605 | CD62L | MEL-14 | Biolegend |
| PE | CD45RA | HI100 | Biolegend |
| BV786 | CD3 | UCHT1 | BD Bioscience |
| BV711 | TIGIT | VSTM3 | Biolegend |
| APC-Cy7 | PD1 | HB3 | Invitrogen |
| PECy7 | aBiot CAR | REA746 | Miltenyi |
| Biotin | CD19 | | Miltenyi |
| BV 510 | TLR2 | 11G7 | BD Bioscience |
| V450 (PB) | HLA-DR | I243 | Biolegend |
| APC | hCD45 | HI30 | Biolegend |
| FITC | CD19 | SJ25C1 | Biolegend |
| AF700 | hCD 17 | 104D2 | Biolegend |
| BV605 | CCR2 | K036C2 | Biolegend |
| PE | CD33 | WM53 | Biolegend |
| BV786 | CD11b | ICRF44 | BD Bioscience |
| BV711 | CD14 | MOP9 | BD Bioscience |
| BV711 | CD15 | W6D3 | BD Bioscience |
| APC-Cy7 | CD34 | 581 | Biolegend |
| PECy7 | VEGFR2 | A16085H | Biolegend |

### References

1. Koh MY, Spivak-Kroizman T, Venturini S, Welsh S, Williams RR, Kirkpatrick DL, and Powis G. Molecular mechanisms for the activity of PX-478, an antitumor inhibitor of the hypoxia-inducible factor-1alpha. Mol Cancer Ther. 2008;7(1):90-100.
2. Zhu AX, Kang YK, Yen CJ, Finn RS, Galle PR, Llovet JM, Assenat E, Brandi G, Pracht M, Lim HY, Rau KM, Motomura K, Ohno I, Merle P, Daniele B, Shin DB, Gerken G, Borg C, Hiriart JB, Okusaka T, Morimoto M, Hsu Y, Abada PB, and Kudo M. Ramucirumab after sorafenib in patients with advanced hepatocellular carcinoma and increased α-fetoprotein concentrations (REACH-2): a randomised, double-blind, placebo-controlled, phase 3 trial. Lancet Oncol. 2019;20(2):282-96.
3. Furie R, Khamashta M, Merrill JT, Werth VP, Kalunian K, Brohawn P, Illei GG, Drappa J, Wang L, and Yoo S. Anifrolumab, an Anti-Interferon-α Receptor Monoclonal Antibody, in Moderate-to-Severe Systemic Lupus Erythematosus. Arthritis Rheumatol. 2017;69(2):376-86.
4. Locatelli F, Jordan MB, Allen C, Cesaro S, Rizzari C, Rao A, Degar B, Garrington TP, Sevilla J, Putti MC, Fagioli F, Ahlmann M, Dapena Diaz JL, Henry M, De Benedetti F, Grom A, Lapeyre G, Jacqmin P, Ballabio M, and de Min C. Emapalumab in Children with Primary Hemophagocytic Lymphohistiocytosis. N Engl J Med. 2020;382(19):1811-22.
5. Magnani CF, Gaipa G, Lussana F, Belotti D, Gritti G, Napolitano S, Matera G, Cabiati B, Buracchi C, Borleri G, Fazio G, Zaninelli S, Tettamanti S, Cesana S, Colombo V, Quaroni M, Cazzaniga G, Rovelli A, Biagi E, Galimberti S, Calabria A, Benedicenti F, Montini E, Ferrari S, Introna M, Balduzzi A, Valsecchi MG, Dastoli G, Rambaldi A, and Biondi A. Sleeping Beauty-engineered CAR T cells achieve antileukemic activity without severe toxicities. J Clin Invest. 2020;130(11):6021-33.
6. Witkowski MT, Dolgalev I, Evensen NA, Ma C, Chambers T, Roberts KG, Sreeram S, Dai Y, Tikhonova AN, Lasry A, Qu C, Pei D, Cheng C, Robbins GA, Pierro J, Selvaraj S, Mezzano V, Daves M, Lupo PJ, Scheurer ME, Loomis CA, Mullighan CG, Chen W, Rabin KR, Tsirigos A, Carroll WL, and Aifantis I. Extensive Remodeling of the Immune Microenvironment in B Cell Acute Lymphoblastic Leukemia. Cancer Cell. 2020;37(6):867-82.e12.
7. Liberzon A, Birger C, Thorvaldsdóttir H, Ghandi M, Mesirov JP, and Tamayo P. The Molecular Signatures Database (MSigDB) hallmark gene set collection. Cell Syst. 2015;1(6):417-25.
8. Wu Y, Yang S, Ma J, Chen Z, Song G, Rao D, Cheng Y, Huang S, Liu Y, Jiang S, Liu J, Huang X, Wang X, Qiu S, Xu J, Xi R, Bai F, Zhou J, Fan J, Zhang X, and Gao Q. Spatiotemporal Immune Landscape of Colorectal Cancer Liver Metastasis at Single-Cell Level. Cancer Discov. 2022;12(1):134-53.
9. Bleve A, Durante B, Sica A, and Consonni FM. Lipid Metabolism and Cancer Immunotherapy: Immunosuppressive Myeloid Cells at the Crossroad. Int J Mol Sci. 2020;21(16).
10. Chun E, Lavoie S, Michaud M, Gallini CA, Kim J, Soucy G, Odze R, Glickman JN, and Garrett WS. CCL2 Promotes Colorectal Carcinogenesis by Enhancing Polymorphonuclear Myeloid-Derived Suppressor Cell Population and Function. Cell Rep. 2015;12(2):244-57.
11. Shime H, Maruyama A, Yoshida S, Takeda Y, Matsumoto M, and Seya T. Toll-like receptor 2 ligand and interferon-γ suppress anti-tumor T cell responses by enhancing the immunosuppressive activity of monocytic myeloid-derived suppressor cells. Oncoimmunology. 2017;7(1):e1373231.
12. Alshetaiwi H, Pervolarakis N, McIntyre LL, Ma D, Nguyen Q, Rath JA, Nee K, Hernandez G, Evans K, Torosian L, Silva A, Walsh C, and Kessenbrock K. Defining the emergence of myeloid-derived suppressor cells in breast cancer using single-cell transcriptomics. Sci Immunol. 2020;5(44).
13. Pliner HA, Shendure J, and Trapnell C. Supervised classification enables rapid annotation of cell atlases. Nat Methods. 2019;16(10):983-6.
14. Bengsch B, Johnson AL, Kurachi M, Odorizzi PM, Pauken KE, Attanasio J, Stelekati E, McLane LM, Paley MA, Delgoffe GM, and Wherry EJ. Bioenergetic Insufficiencies Due to Metabolic Alterations Regulated by the Inhibitory Receptor PD-1 Are an Early Driver of CD8(+) T Cell Exhaustion. Immunity. 2016;45(2):358-73.
15. Chapman NM, and Chi H. Metabolic adaptation of lymphocytes in immunity and disease. Immunity. 2022;55(1):14-30.
16. Milner JJ, Toma C, He Z, Kurd NS, Nguyen QP, McDonald B, Quezada L, Widjaja CE, Witherden DA, Crowl JT, Shaw LA, Yeo GW, Chang JT, Omilusik KD, and Goldrath AW. Heterogenous Populations of Tissue-Resident CD8(+) T Cells Are Generated in Response to Infection and Malignancy. Immunity. 2020;52(5):808-24.e7.
17. Siddiqui I, Schaeuble K, Chennupati V, Fuertes Marraco SA, Calderon-Copete S, Pais Ferreira D, Carmona SJ, Scarpellino L, Gfeller D, Pradervand S, Luther SA, Speiser DE, and Held W. Intratumoral Tcf1(+)PD-1(+)CD8(+) T Cells with Stem-like Properties Promote Tumor Control in Response to Vaccination and Checkpoint Blockade Immunotherapy. Immunity. 2019;50(1):195-211.e10.
18. Chen Z, Ji Z, Ngiow SF, Manne S, Cai Z, Huang AC, Johnson J, Staupe RP, Bengsch B, Xu C, Yu S, Kurachi M, Herati RS, Vella LA, Baxter AE, Wu JE, Khan O, Beltra JC, Giles JR, Stelekati E, McLane LM, Lau CW, Yang X, Berger SL, Vahedi G, Ji H, and Wherry EJ. TCF-1-Centered Transcriptional Network Drives an Effector versus Exhausted CD8 T Cell-Fate Decision. Immunity. 2019;51(5):840-55.e5.
19. Mantovani A, Allavena P, Sica A, and Balkwill F. Cancer-related inflammation. Nature. 2008;454(7203):436-44.
20. Virgin HW, Wherry EJ, and Ahmed R. Redefining chronic viral infection. Cell. 2009;138(1):30-50.
21. Browaeys R, Saelens W, and Saeys Y. NicheNet: modeling intercellular communication by linking ligands to target genes. Nat Methods. 2020;17(2):159-62.
22. Chen J, López-Moyado IF, Seo H, Lio CJ, Hempleman LJ, Sekiya T, Yoshimura A, Scott-Browne JP, and Rao A. NR4A transcription factors limit CAR T cell function in solid tumours. Nature. 2019;567(7749):530-4.
23. Sumida TS, Dulberg S, Schupp JC, Lincoln MR, Stillwell HA, Axisa P-P, Comi M, Unterman A, Kaminski N, Madi A, Kuchroo VK, and Hafler DA. Type I interferon transcriptional network regulates expression of coinhibitory receptors in human T cells. Nature Immunology. 2022;23(4):632-42.
24. Magnani CF, Myburgh R, Brunn S, Chambovey M, Ponzo M, Volta L, Manfredi F, Pellegrino C, Pascolo S, Miskey C, Ivics Z, Shizuru JA, Neri D, and Manz MG. Anti-CD117 CAR T cells incorporating a safety switch eradicate human acute myeloid leukemia and hematopoietic stem cells. Mol Ther Oncolytics. 2023;30:56-71.
25. Norelli M, Camisa B, Barbiera G, Falcone L, Purevdorj A, Genua M, Sanvito F, Ponzoni M, Doglioni C, Cristofori P, Traversari C, Bordignon C, Ciceri F, Ostuni R, Bonini C, Casucci M, and Bondanza A. Monocyte-derived IL-1 and IL-6 are differentially required for cytokine-release syndrome and neurotoxicity due to CAR T cells. Nat Med. 2018;24(6):739-48.
26. Magnani CF, Mezzanotte C, Cappuzzello C, Bardini M, Tettamanti S, Fazio G, Cooper LJN, Dastoli G, Cazzaniga G, Biondi A, and Biagi E. Preclinical Efficacy and Safety of CD19CAR Cytokine-Induced Killer Cells Transfected with Sleeping Beauty Transposon for the Treatment of Acute Lymphoblastic Leukemia. Hum Gene Ther. 2018;29(5):602-13.

## Claims

1. An HIF1α or VEGFR2 or IFN inhibitor, or a combination thereof, for use in the treatment of tumor by means of CAR T cells, wherein said inhibitors or combinations thereof enhance CAR T-cell activity by preventing T-cell exhaustion and by reducing the immunosuppressive effect of tumor microenvironment.

2. A combination of inhibitors for use according to claim 1, which is selected from the group consisting of (i) HIF1α and VEGFR2 inhibitors and (ii) HIF1α and IFN inhibitors.

3. An inhibitor or inhibitor combination for use according to claim 1, wherein said inhibitor is an antibody.

4. The inhibitor or inhibitor combination for use according to claim 1, wherein said tumor is a hematologic tumor.

5. The inhibitor or inhibitor combination for use according to claim 4, wherein said hematologic tumor is acute lymphoblastic leukemia.

6. The inhibitor or inhibitor combination for use according to claim 1, wherein said inhibitor or inhibitor combination is administered immediately before, during or immediately after the administration of the CAR T cell therapy.

7. The inhibitor or inhibitor combination for use according to claim 1, wherein said inhibitor or inhibitor combination is administered prior to, following or concomitantly with (i) lymphodepletion or (ii) surgery to remove a tumor or (iii) radiotherapy.

8. The inhibitor or inhibitor combination for use according to claim 1, wherein said tumor expresses CD19 and said CAR T cells carry a CD19-specific CAR.

9. A method of predicting the response to CAR T-cell therapy, which comprises determining, in a sample of T cells isolated from a subject undergoing CAR T-cell therapy, the expression of a cell surface marker selected from PD1, TIGIT, TIM3 and LAG3, or a combination thereof, wherein detection of said surface marker expression in the T cell sample is indicative of a poor response to the CAR T-cell therapy.

10. The method of claim 8, wherein the determination of surface marker expression is carried out by means of marker-specific antibodies.
